(19) Europäisches Patentamt
European Patent Office
Office européen des brevets

(11) Veröffentlichungsnummer: **0 004 913**
**B1**

(12) # EUROPÄISCHE PATENTSCHRIFT

(45) Veröffentlichungstag der Patentschrift: **13.06.84**

(21) Anmeldenummer: **79101036.6**

(22) Anmeldetag: **05.04.79**

(51) Int. Cl.³: **C 07 J 9/00**, C 12 N 15/00, C 12 N 1/20//C12P33/16

(54) **Verfahren zum selektiven Seitenkettenabbau von Steroidverbindungen und zur Herstellung hierzu geeigneter Mikroorganismen-Defektblockmutanten sowie neue Mikroorganismenstämme (I).**

(30) Priorität: **17.04.78 AT 2662/78**

(43) Veröffentlichungstag der Anmeldung:
**31.10.79 Patentblatt 79/22**

(45) Bekanntmachung des Hinweises auf die Patenterteilung:
**13.06.84 Patentblatt 84/24**

(84) Benannte Vertragsstaaten:
**BE DE FR GB IT NL SE**

(56) Entgegenhaltungen:
**US - A - 4 029 549**

**AGRICULTURAL AND BIOLOGICAL CHEMISTRY, B. 42/2), Februar 1978, Tokyo, JP, KEI ARIMA et al.: "Microbial production of 3-oxobisnorchola-1,4-dien 22-oic acid", Seiten 411-416**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Hill, Frank, Dr.**
**Schlesienstrasse 23**
**D-4020 Mettmann-Obschwarzbach (DE)**
Erfinder: **Preuss, Wolfgang, Dr.**
**Finkenweg 12**
**D-4019 Monheim Rhld. (DE)**
Erfinder: **Schindler, Joachim, Dr.**
**Am Eichelkamp 158**
**D-4010 Hilden Rhld (DE)**
Erfinder: **Schmid, Rolf, Dr.**
**Am Nettchesfeld 30**
**D-4000 Düsseldorf 13 (DE)**
Erfinder: **Struve, Alfred, Dr.**
**Am Eichelkamp 53**
**D-4010 Hilden Rhld. (DE)**

(74) Vertreter: **Werner, Hans-Karsten, Dr. et al,**
**Deichmannhaus am Hauptbahnhof**
**D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 913 B1

(56) Entgegenhaltungen:
CHEMICAL ABSTRACTS, Band 87, Nr. 23, 5. Dezember 1977, Columbus, Ohio, USA, KEI ARIMA et al.: "Microbial production of 3-oxobisnorchola-1,4-dienic acid", Seite 464, linke Spalte, Zusammenfassung Nr. 182618j

CHEMICAL ABSTRACTS, Band 86, Nr. 3, 17. Januar 1977, Columbus, Ohio, USA, C.K.A. MARTIN et al.: "Microbial transformation of beta-sitosterol by Nocardia sp. M 29", Seite 304, rechte Spalte, Zusammenfassung Nr. 15144z

**Beschreibung**

Der erste Bericht zur mikrobiellen Umwandlung von Steroidverbindungen geht auf 1937 zurück, als Mamoli und Vercellone (Ber. 70, 470 und Ber. 70, 2079) die stereospezifische Reduktion von 17-Ketosteroiden zu 17-$\beta$-hydroxysteroiden beschrieben. Im Jahr 1952 beschrieben Peterson und Murray (US—PS 2 602 769) ein technisch bedeutungsvolles Verfahren zur 11-$\alpha$-Hydroxylierung von Progesteron durch Rhizopus nigricans.

Seit dieser Zeit sind zahlreiche Umwandlungen von Steroidverbindungen mittels Mikroorganismen beschrieben worden (siehe beispielsweise W. Charney und H. L. Herzog, Microbial Transformation of Steroids, Academic Press, New York, 1967).

Trotz dieser zahlreichen Vorschläge zur mikrobiellen Umwandlung von Steroidverbindungen sind nur wenige dieser Reaktionen zur Herstellung wichtiger Steroidhormone aus Ausgangsmaterialien geeignet, die leicht zugänglich sind und in großen Mengen zur Verfügung stehen. Hier sind in erster Linie die in 17-C-Alkylseitenketten enthaltenen Sterinverbindungen pflanzlichen oder tierischen Ursprungs zu nennen, beispielsweise Sterine der Cholestan-, Campestan- und Stigmastan-Serien.

Es ist bekannt, daß zahlreiche Mikroorganismenstämme, beispielsweise solche von Achromobacter, Arthrobacter, Bacillus, Brevibacterium, Corynebacterium, Flavobacterium, Microbacterium, Mycobacterium, Nocardia, Protaminobacter, Serratia und Streptomyces, befähigt sind, auf Sterinverbindungen der genannten Art, z.B. auf Cholesterin, als Kohlenstoffquelle zu wachsen (siehe z.B. Arima et al., 1969, Agr. Biol. Chem. 33: 1636—1643). Dabei werden in der Regel in nicht spezifischer Weise sowohl das Ringsystem als auch eventuell vorhandene Seitenketten am Ringsystem durch die Mikroorganismen angegriffen bzw. bei ihrem Wachstum abgebaut. Überwiegend sind dabei Angriff und Abbau im Ringsystem die bevorzugte und/oder schnellere Reaktion. In einer kürzlich erschienenen Arbeit von Arima et al., Agric. Biol. Chem. 42(2), 411—416 wird über Arbeiten zur Transformierung von Cholesterin durch mikrobiellen Seitenkettenabbau in Gegenwart von Inhibitoren zur Hemmung des Ringabbaus im Steroidgerüst berichtet. Gemäß diesen Angaben war ein beachtlicher Anteil der getesteten ca. 200 Wildstämme in der Lage, unter dem Einfluß der Inhibitoren zu einem weitgehend selektiven Seitenkettenabbau in C—17 des Einsatzmaterials zu führen. Einige Stämme führen dabei zu 3-Oxo-pregna-1,4-dien-20-carbonsäure ($\Delta$-1,4 BNC) in wechselnden Ausbeuten.

Ein entsprechender Hinweis findet sich in Chemical Abstracts, Band 87 (1977), S. 464, linke Spalte. Beschrieben wird hier die Herstellung von $\Delta$-1,4 BNC aus Sterinverbindungen mittels Mikroorganismen durch Seitenkettenabbau in Gegenwart von Komplexbildnern für Eisen, Kupfer, Nickel oder Kobalt. Insbesondere wird $\alpha,\alpha'$-Dipyridyl als Inhibitor zur Hemmung des Ringabbaus im Steroidgerüst eingesetzt. Es gelingt auf diese Weise, mit Nocardia corallina IFO 3338 $\Delta$-1,4-BNC in geringer Menge zu isolieren. Eine mikrobielle Transformation von $\beta$-Sitosterin durch Nocardia sp. M 29 wird in Chemical Abstracts, Band 86 (1977), S.304, rechte Spalte, beschrieben. Durch Inhibierung des die Steroidringstruktur öffnenden Enzyms mittels $\alpha,\alpha'$-Dipyridyl gelingt es, aus Sitosterin insgesamt geringe Ausbeuten an dem 4-en-3,17-dion und dem 1,4-Dien-3,17-dion herzustellen. Der Seitenkettensubstituent in 17-Stellung ist damit also vollständig abgebaut, stattdessen liegt im Abbauprodukt in 17-Stellung eine Oxogruppe vor.

Aufgrund der potentiellen Bedeutung der in grossen Mengen zur Verfügung stehenden natürlichen Sterinverbindungen mit 17-C-Seitenketten pflanzlichen Ursprungs (Phytosterine) oder tierischen Ursprungs (Cholesterin) als Ausgangsmaterial für hochwertige Pharmazeutika mit Steroidgrundstruktur sind zahlreiche Versuche unternommen worden, zum selektiven Seitenkettenabbau auf mikrobiologischen Wege zu kommen. Eine zusammenfassende Übersicht dieser Arbeiten findet sich in Adv. Appl. Microbiol. *22*, 29 (1977), 29—58, Christoph K. A. Martin "Microbial Cleavage of Sterol Side Chains". Verwiesen wird insbesondere auf das Kapitel IV dieser Veröffentlichung, das die drei bisher eingeschlagenen Wege zum selektiven Seitenkettenabbau unter A bis C aufschlüsselt. Die Vorschläge des Standes der Technik fallen demgemäß in die folgenden drei Gruppen: Umwandlung der Ringstruktur der Sterinausgangsverbindungen derart, daß der Mechanismus der Ringspaltung unterbunden und damit der selektive Seitenkettenabbau möglich wird. Mitverwendung von Inhibitoren zur Hemmung des Steroidringabbaus und/oder des Mikroorganismenwachstums sowie schließlich die Suche nach Mikroorganismenmutanten, die zum gewünschten möglichst weitgehend selektiven Seitenkettenabbau führen.

Überwiegend werden nach diesem Verfahren des Standes der Technik als Endprodukt des mikrobiellen Abbaus 17-Ketosteroidverbindungen erhalten, die zwar als Zwischenprodukte zur technischen Herstellung von Hormonen der Estran-Androstan- und Spirostan-Serien wertvoll sind, die jedoch weniger geeignet sind für die Herstellung von Steroidhormonen der Pregnanserien, beispielsweise Progesteron, Hydrocortison, Cortison, Prednison, Prednisolon, Triamcinolon und dergleichen. In den zuletzt genannten Fällen müssen in 17-Stellung stereospezifische Seitenketten chemisch wieder eingebaut werden.

Nur gelegentlich ist es bisher gelungen, als Stoffwechselprodukte des mikrobiellen Abbaus Steroidverbindungen zu erhalten, die in 17-C-Stellung einen Alkylseitenkettenrest und dabei insbesondere den Rest der $\alpha$-Propion säure enthalten. Steroidverbindungen dieser Art wären insbesondere für die großtechnische Gewinnung von Steroidhormonen der Pregnanserie aus Sterinen natürlichen

Ursprungs geeignet. Es ist dabei einleuchtend, daß die Gewinnung von partiellen Sterinabbauprodukten dieser Art deswegen besonders schwierig ist, weil hier ja nicht nur der selektive Seitenkettenabbau—unter gleichzeitiger Verhinderung des Aufbrechens und Abbaus der Ringstruktur—notwendig ist, sondern zusätzlich auch noch das Wachstum der Mikroorganismen unter Seitenkettenabbau in einem Stadium zum Abbruch kommen muß, der an sich nach bischerigen Erkenntnissen lediglich Durchgangsstation bis zum Erreichen der 17-Ketosteroidstruktur ist. J. M. Whitmarsh beschreibt in "The Biochemical Journal", 90, 1964, 23 p bis 24 p, den mikrobiologischen Abbau von Cholesterin mit einer Norcardia-Kultur in Gegenwart von Inhibitoren, wie 8-Hydroxychinolin unter Bildung geringer Mengen von 3-Oxo-pregna-4-en-20-carbonsäure (Δ-4 BNC) und 3-Oxo-pregna-1,4-dien-20-carbonsäure (Δ-1,4 BNC). Die kürzlich erschienene US—PS 4 029 549 (Antosz et al.) beschreibt die Mikroorganismenmutante NRRL B—8119, mit der es möglich sein soll, selektiv 17-C-Steroidverbindungen mit 8 bis 10 Kohlenstoffatomen im 17-C-Alkylrest zu 9$\alpha$-OH AD und 9$\alpha$-OH BN-Säure abzubauen. Wie Jedoch insbesondere das Kapitel IV C aus der Veröffentlichung C. K. A. Martin a.a.O., Seiten 50 bis 52, zeigt, gibt es bis heute keinen zuverlässigen Weg der mikrobiologischen Herstellung von 17-C-Steroid-$\alpha$-propionsäureverbindungen aus 17-C-Seitenketten-Steroidsubstraten bzw. zur reproduzierbaren Gewinnung von Mikroorganismen-Defektblockmutanten, die in zuverlässiger Weise und in hohen Ausbeuten selbst dann 17-C-Steroid-$\alpha$-propionsäureverbindungen als Endprodukt des enzymatischen Abbaus liefern können, wenn in Abwesenheit von Inhibitoren der genannten Art gearbeitet werden soll. Das Arbeiten in Abwesenheit oder in Gegenwart nur geringer Mengen der Inhibitoren muß aber aus technischen Gründen—z.B. wegen der unerwünschten hohen Raum-Zeit-Ausbeuten—wünschenswert erscheinen.

Die Erfindung hat sich die Aufgabe gestellt, die Herstellung von 17-C-Steroid-$\alpha$-propionsäureverbindungen in technisch brauchbaren und verbesserten Ausbeuten durch selektiven mikrobiellen Abbau von 17-C-Seitenketten-Steroidsubstraten zu ermöglichen. Dabei sollen in gezielter Weise hergestellte und ausgewählte Mikroorganismen-Defektblockmutanten zum Einsatz kommen, die aus geeigneten Wildstämmen zuvor gewonnen worden sind. Es soll dabei insbesondere möglich-wenn auch keineswegs zwingend—sein, mit den ausgewählten Defektblockmutanten auch in Abwesentheit von den Steroidringabbau und/oder das Wachstum von Mikroorganismen hemmenden Inhibitoren zu arbeiten. Eine weitere Aufgabe der Erfindung ist die sichere und reproduzierbare Herstellung der Mikroorganismen-Defektblockmutanten, die zur verbesserten, insbesondere inhibitorfreien technischen Gewinnung von 17-C-Steroid-$\alpha$-propionsäureverbindungen aus Sterinen natürlichen Ursprungs geeignet sind.

Im engeren Sinne hat sich dabei die Erfindung insbesondere die mikrobielle Herstellung von 3-Oxo-pregna-4-en-20-carbonsäure (Δ-4 BNC) und/oder 3-Oxo-pregna-1,4-dien-20-carbonsäure (Δ-1,4 BNC) auf dem beschriebenen Wege zur Aufgabe gestellt. Ausgangsmaterial für die mikrobioligische Herstellung dieser Verbindungen sollen insbesondere natürliche und/oder pflanzliche Sterine sein, die bisher als Abfallprodukte kaum praktische Bedeutung gewonnen haben. Auch einfache Abkömmlinge dieser natürlichen Ausgangsmaterialien sollen für die Erfindung geeignet sein.

In einer besondere Ausführungsform soll die Erfindung insbesondere Wege schaffen, hochwirksame Mikroorganismen-Defektblockmutanten für das jeweils im technischen Verfahren eingesetzte bestimmte Sterinausgangsmaterial zu schaffen und damit die Anpassung des Sterineinsatzmaterials und der zu wählenden Mikroorganismen-Defektblockmutanten zu ermöglichen. Die erfindungsgemäßen Verfahrensprodukte Δ-4 BNC und Δ-1,4 BNC sind wertvolle Zwischenprodukte beispielsweise für die Herstellung von Verbindungen der Progesteron-Serie.

Die Strukturformeln der hier angegebenen Verbindungen Δ-4 BNC und Δ-1,4 BNC sind die folgenden:

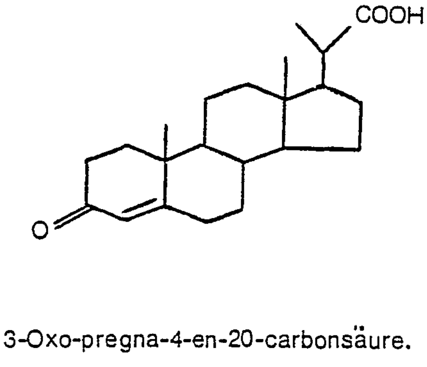

(Δ—4 BNC)

3-Oxo-pregna-4-en-20-carbonsäure.

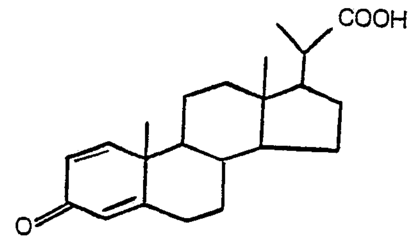

(Δ–1,4 BNC)

3-Oxo-pregna-1,4-dien-20-carbonsäure.

In einer ersten Ausführungsform betrifft die Erfindung ein Verfahren zur Herstellung von aerob arbeitenden Mikroorganismen-Defektblockmutanten, die zur technischen Gewinnung von 17-C-Steroid-$\alpha$-propionsäureverbindungen, insbesondere zur Herstellung von 3-Oxo-pregna-4-en-20-carbonsäure und/oder 3-Oxo-pregna-1,4-dien-20-carbonsäure aus 17-C-Seitenketten-Steroidsubstraten, z.B. aus Sterinverbindungen tierischen oder pflanzlichen Ursprungs, durch wenigstens weitgehend selektiven Seitenkettenabbau, gewünschtenfalls auch in Abwesenheit von den Steroidringabbau und/oder das Mikroorganismenwachstum hemmenden Inhibitoren befähigt sind, wobei dieses Verfahren dadurch gekennzeichnet ist, daß man.

1) einen Mikroorganismen-Wildstamm isoliert und züchtet, der zum aeroben Wachstum auf Sterinverbindungen als alleiniger Kohlenstoffquelle befähigt ist und dabei den 17-C-Seitenkettenabbau einem Ringabbau genenüber bevorzugt,

2) den Wildstamm einer an sich bekannten Mutationsbehandlung unterwirft.

3) die Mutantenpopulation auf einem Trennmedium (Anreicherungsmedium für die gewünschten Mutanten) züchtet, auf dem die 17-C-Steroid-$\alpha$-propionsäureverbindungen liefernden Mutanten nicht oder praktisch nicht wachsen, während die unerwünschten begleitenden Mutantenstämme wachsen und hierdurch bzw. während ihres Wachstums abgetötet werden und

4) den verbliebenen Rest der Mutantenstämme auf 17-C-Seitenkettensterinen züchtet und dabei die Stämme mit optimaler Produktion der 17-C-Steroid-$\alpha$-propionsäureverbindungen isoliert.

Gegenstand der Erfindung sind weiterhin die nach diesem Verfahren hergestellten und dabei insbesondere Δ-4 BNC und/oder Δ-1,4 BNC liefernden Defektblockmutanten. Insbesondere betrifft die Erfindung in diesem Zusammenhang eine neue Defektblockmutante, die beim Centraalbureau voor Schimmelcultures, Baarn, Niederlande, unter der Nummer CBS 437.77 hinterlegt worden ist. Diese neue Blockmutante ist zur inhibitorfreien Herstellung von 3-Oxo-pregna-4-en-20-carbonsäure oder 3-Oxo-pregna-1,4-dien-20-carbonsäure auch in Abwesenheit der genannten Inhibitoren aus Sterinen natürlichen oder pflanzlichen Ursprungs geeignet. Sie wird nach dem erfindungsgemäßen Verfahren aus dem ebenfalls von der Anmelderin erstmals isolierten, beim Centraalbureau voor Schimmelcultures, Baarn, Niederlande, unter der Nummer CBS 660.77 hinterlegten neuen Windstamm gewonnen, der zu der Gruppe der coryneformen Bakterien gehörig ist.

Insbesondere ist schließlich Gegenstand der Erfindung ein Verfahren zur Herstellung von 3-Oxo-pregna-4-en-20-carbonsäure (Δ-4 BNC) und/oder 3-Oxo-pregna-1,4-dien-20-carbonsäure (Δ-1,4 BNC) durch mikrobiellen Seitenkettenabbau an 17-C-Seitenketten-Steroidsubstraten, das dadurch gekennzeichnet ist, daß man

a) einen auch in Abwesenheit von Inhibitoren zur Hemmung des Steroidringabbaus und/oder des Mikroorganismenwachstums Δ-4 BNC und/oder Δ-1,4 BNC liefernden Mikroorganismus in einem wäßrigen Nährmedium unter aeroben Bedingungen und in Gegenwart eines 17-C-Seitenketten-Steroidsubstrats unter Anreicherung von 3-Oxo-pregna-4-en-20-carbonsäure und/oder 3-Oxo-pregna-1,4-dien-20-carbonsäure züchtet und

b) anschließend die gebildete 3-Oxo-pregna-4-en-20-carbonsäure und/oder 3-Oxo-pregna-1,4-dien-20-carbonsäure isoliert. Die Ausbeute an Δ-4 BNC und/oder Δ-1,4 BNC soll dabei mindestens 15 Gew.-%—bezogen auf eingesetztes Steroidsubstrat—betragen.

Die Gewinnung der Mikroorganismen-Defektblockmutanten gemäß der Erfindung.

Ein entscheidendes Gegenstand der Erfindung liegt in der wohl erstmaligen Schaffung eines sicher reproduzierbaren Verfahrens zur Gewinnung von Defektblockmutanten der geschilderten Art, d.h. von Mikroorganismen, die durch eine ganz bestimmte Kombination von Selektionen und Mutationen mit anschließender erneuter Selektion aus Wildstämmen erhalten werden können, die auf Steroidverbindungen der geschilderten Art zu wachsen vermögen. Die nach dem erfindungsgemäßen Verfahren erhaltenen Mikroorganismen-Defektblockmutanten zeichnen sich dadurch aus, daß sie einerseits gegenüber einem Wachstum auf der steroidringstruktur unter Verzehr dieser Ringe als Kohlenstoffquelle wenigstens weitgehend blockiert sind, andererseits sind die erfindungsgemäß erhaltenen Mikroorganismenmutanten aber auch gegenüber dem Abbau eines in 17-C-Stellung am Steroid vorliegenden bzw. entstehenden $\alpha$-Propionsäure restes so hinreichend blockiert, daß die gezielte Herstellung der 17-C-Steroid-$\alpha$-propionsäureverbindungen aus entsprechenden Steroidsbustraten mit längeren 17-C-Seitenketten und ihre Isolierung aus der Fermentationsbrühe in guten Ausbeuten technisch möglich wird. Es ist dabei besonders vorteilhaft, daß gewünschtenfalls in Abwesenheit von Inhibitoren der genannten Art—siehe z.B. Christoph K. A. Martin, a.a.O., Kapitel IVB—gearbeitet werden kann. Durch die erfindungsgemäße Kombination von bestimmter Wildstammauswahl und nachfolgender Mutationsund Selektionsschritten wird dieses ermöglicht.

Im erfindungsgemäßen Verfahren zur Gewinnung der gewünschten Blockmutanten reihen sich die folgenden vier Verfahrensstufen aneinander:

Stufe 1: Gezielte Auswahl von Mikroorganismen-Wildstämmen,

Stufe .2: Mutationsbehandlung

Stufe 3: Trennung der gewonnenen Mutantenpopulation aus Stufe 2 bzw. Anreicherung der gewünschten Mutanten aus der insgesamt gewonnenen Mutantenpopulation,

Stufe 4: Züchtung der in Stufe 3 abgetrennten bzw. angereicherten Mutantenstämme und Auswahl der Blockmutantenstämme mit optimaler Produktion des gewünschten 17-C-Steroid-$\alpha$-propionsäure-Verfahrensprodukts.

Diese vier Verfahrensstufen können in einmaligem Durchgang zur Gewinnung der gewünschten Defektblockmutanten durchlaufen werden. Es kann aber auch zweckmäßig sein, die Verfahrensstufen 2 und 3 ein- oder mehrfach derart zu wiederholen, daß die in Stufe 3 abgetrennte—bzw. angereicherte—gewünschte Mutantenpopulation ernaut einer Mutationsbehandlung gemäß Stufe 2 unterworfen und dann wiederum in Stufe 3 aufgetrennt wird.

Im Folgenden wird auf die Einzelheiten der aufgezählten Stufen 1 bis 4 eingegangen.

Stufe 1: Isolierung der geeigneten Wildstämme

Die auszuwählenden Mikroorganismen-Wildstämme sollen befähigt sein, unter aeroben Bedingungen auf Steroidverbindungen und dabei insbesondere auf der Steroidverbindung mit 17-C-Seitenketten zu wachsen. Die für die Erfindung geeigneten Stämme besitzen dabei vorzugsweise wenigstens etwa die gleiche Abbaugeschwindigkeit für die Seitenkette wie für den Ringenteil der Steroidverbindung. Bevorzugt weisen die Wildstämme jedoch eine erhöhte Seitenkettenabbaugeschwindig'kiet im Vergleich mit dem Ringabbau auf.

Die Isolierung der Wildstämme kann dabei in an sich bekannter Weise aus Erdproben erfolgen. Wohl wegen der weiten Verbreitung pflanzlicher und tierischer Sterinverbindungen finden sich geeignete Wildstämme der genannten Art praktisch regelmäßig in Erdproben beliebigen Ursprungs. Ihre Züchtung erfolgt unter aeroben Bedingungen in Gegenwart eines wäßrigen Nährmediums und vorzugsweise in Gegenwart einer Steroidverbindung mit einer 17-C-Seitenkette als einzige Kohlenstoffquelle. Es ist dabei möglich—allerdings keineswegs notwendig—schon an dieser Stelle des erfindungsgemäßen Selektionsverfahrens den Typ von Steroidverbindungen als Kohlenstoffquelle vorzulegen, der letztlich mit den angestrebten Defektblockmutanten abgebaut werden soll. Soll also beispielsweise Cholesterin oder ein Phytosterin letztlich zur 17-C-Steroid-$\alpha$-propionsäureverbindung abgebaut werden, so kann eine dieser natürlichen Sterinverbindungen-gewünschtenfalls die im technischen Verfahren einzusetzende bestimmte Sterinverbindung—als vorzugsweise einzige Kohlenstoffquelle in dem ansonsten konventionellen Nährmedium zur Auswahl und Züchtung des Wildstammes vorliegen. Es hat sich allerdings auch gezeigt, daß es nicht erforderlich sein muß, schon an dieser Stelle eine Abstimmung zwischen Sterin-Kohlenstoffquelle bei der Auswahl und Züchtung des Wildstammes und der letztlich im Steroidabbauverfahren einzusetzenden Sterinverbindung vorzunehmen.

Im Rahmen des Gesamtverfahrens kommt der Auswahl der richtigen Wildstämme eine besondere Bedeutung zu. Es hat sich gezeigt, daß gewöhnlich die Wildstämme überwiegend den Ringabbau gegenüber einem Seitenkettenabbau bevorzugen. Die erfindungsgemäß auszuwählenden Wildstämme sollen wenigstens etwa gleiche Abbaugeschwindigkeiten für die Seitenketten wie für den Ringanteil der Steroidverbindungen aufweisen. Besonders bevorzugt werden jedoch solche Wildstämme ausgewählt und gezüchtet, die beim Wachstum auf Sterinverbindungen mit gesättigten oder ungesättigten Alkylresten in 17-C-Stellung, und zwar vorzugsweise Resten mit 8 bis 10 C-Atomen eine selektive Abbauleistung—gemessen unter den im folgenden beschriebenen Standardbedingungen—gemäß der allgemeinen Formel

$$I = a \cdot 10^{b}$$

ergeben. Hierin ist a der Wachstumsfaktor und bl der Selektivitätsfaktor des Wildstammwachstums wie im folgenden definiert. Der Selektivitätsindex I, der für die Auswahl der geeigneten und der bevorzugten Wildstämme charakteristisch ist, ergibt sich somit als Produkt gemäß der angegebenen Formel aus dem Wachstumsfaktor a und dem Selektivitätsfaktor b. Der Zahlenwert von I für die bevorzugten Mikroorganismen-Wildstämme beträgt wenigstens 10. Höhere Zahlenwerte für I sind unerwünscht und können beispielsweise bei wenigstens 100 liegen. Bei der Selektion von Windstämmen aus einer

größeren isolierten Wildstammpopulation kann es wünschenswert sein, die Stämme mit den höchsten Zahlenwerten für I den weiteren Stufen des erfindungsgemäßen Verfahrens zu unterwerfen. So kann es besonders zweckmäßig sein, solche Wildstämme auszusuchen, für die der Zahlenwert I wenigstens $10^5$ beträgt. Es hat sich im übrigen gezeigt, daß der Absolutwert von I durch die Auswahl des jeweiligen als Kohlenstoffquelle eingesetzten Steroidsubstarats beeinflußt werden kann. So ist es beispielsweise möglich, daß ein ausgewählter Wildstamm bei seiner Züchtung auf einer Sterinverbindung tierischen Ursprungs im hier besprochenen ersten Selektionsschritt wesentlich besser wächst—und dementsprechendeinen höheren Wert für I liefert—als auf einem pflanzlichen Sterin als Kohlenstoffquelle. Unbeschadet hiervon gelten die zuvor angegebenen bevorzugten Bedingungen für die Wildstammauswahl.

Die Bestimmung des Wachstumsfaktors a und des Selektivitätsfaktors b des Wildstammwachstums erfolgt dabei unter den folgenden Standardbedingungen (aerobe Verfahrensführung):

Wachstumsfaktor a:

Der jeweilige Mikroorganismenstamm wird in 500 ml Erlenmeyerkolben (100 ml Nährlösung der im folgenden beschriebenen Zusammensetzung) bei 30°C auf der Schüttelmaschine (Schüttelfrequenz: 150 UpM) inkubiert.

Das Nährmedium hat die folgende Zusammensetzung (pro Liter):

| | |
|---|---|
| 1,0 g | $KH_2PO_4$ |
| 0,5 g | $MgSO_4 \cdot 7\ H_2O$ |
| 0,1 g | NaCl |
| 0,1 g | $CaCl_2 \cdot 2H_2O$ |
| 5 g | $(NH_4)_2SO_4$ |
| 1,0 g | Tween 80 (Polyoxyäthylensorbitanmonooleat) |
| 2,0 g | der 17-C-Seitenkettensterinverbindung, z.B. Cholesterin oder Sitosterin |
| 0,5 g | Hefeextrakt |
| 0,01 g | 1-Histidin |
| 0,02 g | dl-Methionin |
| 0,02 g | dl-Tryptophan |
| 2 $\mu$g | Biotin |
| 400 $\mu$g | Calciumpantothenat |
| 2 $\mu$g | Folsäure |
| 2000 $\mu$g | Inosit |
| 400 $\mu$g | Niacin |
| 200 $\mu$g | p-Aminobenzoesäure |
| 400 $\mu$g | Pyridoxinhydrochlorid |
| 200 $\mu$g | Riboflavin |
| 400 $\mu$g | Thiaminhydrochlorid |

Spurenelementlösung

pH-Wert des Nährmediums 6,8.

Zur Messung des Wachstums (Zelldichte) werden in zeitlichen Abständen den Schüttelkulturen aliquote Mengen Zellsuspension entnommen und die jeweilige Extinktion (E') im Zeiss-Photometer (Typ PL 4) bei einer Wellenlänge von 620 nm (Schichtdicke d—1 cm) gegen destilliertes Wasser gemessen. Die Ausgangsextinktion $E_0$ beträgt vor Beginn des Wachstums—d.h. zum Zeitpunkt $t_0$-etwa 0,775. Die Suspensionen werden jeweils soweit verdünnt, daß im Bereich $E' = 0,7$ gemessen werden kann.

Der Wachstumsfaktor a ergibt sich als die maximale optische Dichte der Zellsuspension am Ende der logarithmischen Wachstumsphase (Zeitpunkt $t_1$, Extinktion $E_1$), die unter den gegebenen Kulturbedingungen nach spätestens 5 Tagen erreicht wird, d.h. $a = \Delta E = E_1 — E_0$.

Selektivitätsfaktor b:

Die zu prüfenden Stämme werden in Nährbouillon I (Merck) plus 0,1% Tween 80 (Polyoxyäthylensorbitanmonooleat) plus 0,1% Sterinverbindung, z.B. Cholesterin, 48 Stunden bei 30°C angezüchtet, die Zellen anschließend mit Puffer gewaschen und in Phosphat-Puffer ($p_H$ 7,5) resuspendiert. Der Abbau der Sterinverbindung wird in Parallelansätzen mit radioaktiv markierten Sterinverbindungen, z.B. 4—14 C- und 26—14 C-Cholesterin oder entsprechend radioaktiv markierten Sitosterinverbindungen in Warburg-Gefäßen gemessen.

Zu 2,4 ml Zellsuspension im Hauptgefäß werden 0,2 ml Sterinlösung (z.B. Cholesterinlösung) gegeben (5 $\mu$ Mol mit ca. 0,1 $\mu$Ci markiertem Substrat in 0,1%iger Tween 80-Lösung), der Ansatz 6 Stunden bei 30°C inkubiert und die Reaktion anschließend durch Zugabe von 0,2 ml 4n $H_2So_4$ gestoppt.

Das bei der Substratspaltung entstehende aktive $CO_2$ wird in Äthanolamin (0,2 ml) aufgefangen und im Scintillationscounter gemessen (0,1 ml Proben in jeweils 15 ml Scintillatarflüssigkeit "Unisolv I", Fa. Zinsser).

Der Selektivitätsfaktor b ist das dimensionslose Verhältnis der so gemessenen Radioaktivität

$$b = \frac{^{14}CO_2 \text{ aus Seitenkette von } (26\text{-}^{14}C)\text{-Sterinverbindung}}{^{14}CO_2 \text{ aus Ringsystem von } (4\text{-}^{14}C)\text{-Sterinverbindung}}$$

In dieser Gleichung bedeutet der Begriff "Sterinverbindung" die jeweils eingesetzte radioaktiv markierte Sterinverbindung, also beispielsweise entsprechende Cholesterin- oder $\beta$-Sitosterin-Verbindungen.

Die Technik der Bestimmung des mikrobioligischen Abbaus von Sterinverbindungen durch radioaktive Markierung entweder eines Ringkohlenstoffatoms oder eines Seitenkettenkohlenstoffatoms ist im übrigen eine im Stand der Technik bekannte Methodik, auf deren Einzelheiten hier Bezug genommen wird. Verwiesen wird beispielsweise auf die folgenden Veröffentlichungen: Steroids, 677—688 (1964), G. E. Peterson und J. R. Davis "Cholesterol Utilization by Streptomyces spp." sowie J.B.C. *206*, 511—523 (1954) Thressa C. Stadtman et al., "Studies on the Microbioligical Degradation of Cholesterol".

Für die Auswahl der Wildstämme in dieser Stufe 1 des erfindungsgemäßen Verfahrens ist es weiterhin bevorzugt, daß der Selektivitätsfaktor b der Wildstämme wenigstens 1, vorzugsweise wenigstens 2, beträgt. Der Wachstumsfaktor a der Wildstämme sollte wenigstens 0,1 vorzugsweise wenigstens 0,2 betragen und beträgt insbesondere ebenfalls wenigstens 1.

Bei der Auswahl der für die weiteren Stufen des erfindungsgemäßen Verfahrens am besten geeigneten Wildstämme kann es zweckmäßig sein, die Fraktoren a und b gegeneinander abzuwägen. So kann es im Rahmen der Erfindung zweckmäßig sein, Wildstämmen mit einem besonders hohen Selektivitätsfaktor b den Vorzug zu geben und bei der Wahl zwischen verschiedenen isolierten Wildstämmen zunächst nach diesem Selektivitätsfaktor b bei möglichst hohen Zahlenwerten für b auszuwählen. Umgekehrt kann natürlich auch ein besonders gutes wachstum—ausgedrückt durch einen besonders hohen Zahlenwert für den Wachstumsfaktor a—Anlaß sein, für die anschließende Mutation und darauffolgende Selektion der Mutantenpopulation einem solchen Wildstamm den Vorzug zu geben.

Als Wildstämme in Stufe 1 kommen beispielsweise solche von Achromobacter, Arthrobacter, Bacillus, Brevibacterium, Corynebacterium, Flavobacterium, Microbacterium, Mycobacterium, Nocardia, Protaminobacterium, Serratia oder Streptomyces in Frage. Entscheidend fûr das erfindungsgemäße Verfahren ist nicht so sehr die Zugehörigkeit des bestimmten Stammes etwa zu Gattungsbegriffen. Wichtig sind vielmehr seine zuvor diskutierten Eigenschaften bei der Züchtung auf sterinverbindungen enthaltenden Nährmedien und insbesondere sein Selektivitätsindex I, der von dem Wachstumsfaktor a und dem Selektivitätsfaktor b bestimmt wird.

Zu Stufe 2:

Die Mutation des ausgewählten Wildstammes erfolgt in an sich bekannter Weise. Sie kann beispielsweise durch energiereiche Bestrahlung etwa mit UV oder Röntgenstrahlung erfolgen. Geeignet ist insbesondere aber auch die Behandlung der Zellen mit mutagenen Agentien. Geeignete mutagene Agentien sind beispielsweise Nitrosoguanidinverbindungen, wie 1-Methyl-3-nitro-1-nitrosoguaniden oder Äthylmethansulfonat. Im einzelnen kann hier auf die allgemeinen Angaben des Standes der Tecknik verweisen werden, siehe hierzu beispielsweise US—PS 4 029 549, Spalte 2, Zeilen 57 ff., mit den dort enthaltenen Verweisungen.

Grundsätzlich gilt auch für die Erfindung, daß das Ergebnis der Wildstamm-Mutation insoweit unbestimmt ist, als die Eigenschaften der erhaltenen Defektmutanten im einzelnen nicht vorausbestimmbar sind. Trotzdem lassen sich Prinzipien für eine optimale Mutationsauslösung festlegen, da die Gesetze der Statistik auf eine Mikroorganismenpopulation anwendbare sind. Aus der Literatur sind Verfahren bekannt, die optimale Bedingungen für die Induktion von Mutationen zu bestimmen (s.a. E. A. Adelberg, M. Mandel, GCC Chen (1965) "Optimal Conditions for Mutagenisis by N-methyl-N'-nitro-N-nitrosoguanidine in Escherichia coli", Biochem. Biophys. Res. Commun. *18*, 788—795).

Im Rahman des erfindungsgemäßen Verfahrens werden die Bedingungen von Konzentration und menpopulationen die Konzetration und die Einwirkungszeit der mutagenen Agentien so gewählt werden, daß bereits ein Teil der Mikroorganismenpopulation letal geschädigt ist. Dabei steigt in dem überlebenden Teil der Population die Häufigkeit der verschiedensten Mutationen mehr oder weniger stark an.

Im Rahman des erfindungsgemäßen Verfahrens werden die Bedingungen von Konzentration und Einwirkzeit des mutagenen Agenz so gewählt, daß die Ausgangspopulation durch die Behandlung zu 10—99,999% inaktiviert wird. Vorzugsweise wird eine Abtötungsrate von 90—99,99% gewählt. An die Verfahrenstufe 2 der Mutation schließen sich die Verfahrensstufen 3 und 4 an, die erneute Selektionsschritte zur Ausmittelung und Anreicherung der erwünschten Defektblockmutanten zum Gegenstand haben.

Zu Stufe 3:

Zur nachfolgenden Auslese der erfindungsgemäß bestimmt gewünschten Defektblockmutanten aus der großen Population der Mikroorganismen nach der Mutationsbehandlung werden erfindungsgemäß Kulturbedingungen gewählt, unter denen die abgeänderten spezifischen Eigenschaften der entstandenen Mutantenstämme zum Selektionsvorteil werden. Die Anreicherung der erwünschten Defektblockmutanten erfolgt erfindungsgemäß häufig unter Bedingungen, unter denen die spezielle Mutante nicht oder praktisch nicht wächst, während unerwünschte Begleitorganismen wachsen und durch ihr Wachstum oder bei ihrem Wachstum abgetötet werden. Auf diese Weise gelingt es, diejenigen Defektblockmutantenstämme zu isolieren, deren ringabbauende Enzyme blockiert sind, jedoch nach wie vor zum Abbau der 17-C-Seitenkette an der Sterinverbindung befähigt sind, wobei durch weitere Maßnahmen insbesondere dieser Stufe 3 sichergestellt wird, daß gerade solche Defektblockmutanten isoliert werden können, die beim Seitenkettenabbau bevorzugt den $\alpha$-Propionsäurerest in 17-C-Stellung ausbilden.

Hierzu wird in der Verfahrensstufe 3 bid Mutantenpopulation auf einem "Trennmedium" gezüchtet, das letztlich als Anreicherungsmedium für die gewünschten Mutantenstämme dient. Als Mutanten-Trennmedium kann ein wäßriges Nährmedium eingesetzt werden, das als Kohlenstoffquelle eine Steriodverbindung mit einer 17-C-Seitenkette mit nur beschränkter C-Zahl oder auch keine Seitenkette in 17-C-Stellung enthält. Geeignet sind als Kohlenstoffquelle in diesem Mutanten-Trennmedium neben den in 17-C-Stellung nicht substituierten Steroidverbindungen insbesondere solche, die Seitenketten mit bis zu 5 C-Atomen enthalten.

Bevorzugt ist es allerdings, als Kohlenstoffquelle in diesen Mutantentrenn- bzw. Anreicherungsmedium eine Steroidverbindung mit 3 C-Atomen in der 17-C-Seitenkette einzusetzen wobei zweckmäßigerweise hier eine 17-C-Steroid-$\alpha$-propionsäureverbindung oder auch eine Mehrzahl solcher gewünschter 17-C-Steroid-$\alpha$-propionsäureverbindungen als einzige Kohlenstoffquelle verwendet wird.

Es kann dabei weiterhin bevorzugt sein, in diesem Mutantentrennmedium als Kohlenstoffquelle diejenigen 17-C-Steroid-$\alpha$-propionsäureverbindungen einzusetzen, deren Herstellung mittels der erfindungsgemäß gezüchteten Blockmutanten letztendlich angestrebt wird. Soll also im Rahmen der Erfindung aus Sterinen pflanzlichen oder tierischen Ursprungs beispielsweise $\Delta$-4 BNC oder $\Delta$-1,4 BNC letztendlich als Verfahrensprodukt gewonnen werden, so kann es zweckmäßig sein, in dem Mutantentrenn- bzw. An reicherungsmedium dieser Stufe 3 als einzige Kohlenstoff quelle $\Delta$-4 BNC und/oder $\Delta$-1,4 BNC einzusetzen.

Im übrigen wird hier mit konventionellen Nährlösungen unter aeroben bedingungen gearbeitet.

Diejenigen mutierten Mikroorganismen, die aufgrund der Mutationsbehandlung ihre Fähigkeit verloren haben, auf den jetzt vorliegenden Kohlenstoffquellen zu wachsen, können sich beim Bebrüten des Mutantentrennmediums nicht vermehren. Sie wachsem also nicht. Ein anderer Teil der Mutantenpopulation, der entweder bei der Mutationsbehandlung der Stufe 2 nicht hinreichend geschädigt worden ist oder andere Defekte erlitten hat, ist befähigt, auf der Kohlenstoffquelle des Mutantentrennmediums zu wachsen. Bei der Bebrütung tritt hier also eine Vermehrung ein.

Die Erfindung macht sich dieses unterschiedliche Verhalten derart zunutze, daß die Bedingungen im Mutantentrennmedium so gewählt werden, daß die wachsenden Stämme aufgrund ihres Wachstums oder während ihres Wachstums abgetötet werden, ohne daß dabei die nicht wachsenden Mutantenstämme geschädigt werden.

Möglich ist eine solche Trennung beispielsweise durch Zusatz von Antibiotika, beispielsweise durch Zusatz von Penicillinverbindungen. Die Zugabe von Penicillinverbindungen führt zur Abtötung des

Anteils der wachsenden Mikroorganismenstämme, während die nicht wachsenden Stämme ungeschädigt bleiben.

Eine andere Möglichkeit der Mutantentrennung in diesem Stadium ist der Einbau von zellschädigenden, insbesondere radioaktiven, Komponenten in den auf dem Trennmedium wachsenden Anteil der Mutantenpopulation. Geeignet ist beispielsweise der Einbau von $P^{32}$ in die wachsenden Mutantenstämme. Möglich ist das beispielsweise durch Mitverwendung entsprechend radioaktiv markierter Salze in der Nährlösung dieses Selektionsschritts. Für die Trennung mittels $P^{32}$ hat sich insbesondere die Mitverwendung von $NaH_2{}^{32}PO_4$ in der Nährlösung bewährt.

Aus den verbliebenen ungeschädigten Defektblockmutanten können dann z.B. mittels der an sich bekannten Lederbergschen Stempelmethode die erfindungsgemäß angestrebten Defektblockmutantenstämme isoliert werden. Bezüglich der hier eingesetzten Verfahrenstechnik und zur Penicillinanreicherungsmethode und zur Lederbergschen Stempelmethode wird beispielsweise verwiesen auf J. Amer. Chem. Soc. 70, 4267, (1948) Davis, B.D. (Penicillin-Anreicherungsmethode), J. Bact. 63, 399 (1952) Lederberg, J., Lederberg, E. M. (Lederbergsche Stempelmethode).

Zu Stufe 4:

Die so isolierten und selektierten Defektblockmutanten können jetzt auf einem üblichen Nährmedium angezüchtet werden und dann gewünschtenfalls einer weiteren Selektion unterworfen werden. Die Selektion kann hier beispielsweise nach dem angestrebten Ergebnis der Mikroorganismenstämme auf dem beabsichtigten Einsatzmaterial, beispielsweise also auf natürlichen oder pflanzlichen Sterinverbindungen erfolgen, wobei hier insbesondere die chemische Natur der Stoffwechselprodukte des Mikroorganismen-Wachstums und die Wachstumsfreudigkeit der Stämme bestimmend sein können. Naturgemäß wird man Blockmutanten mit optimaler Produktivität des letztlich gewünschten Verfahrensprodukts bevorzugen. Diese Stämme können dann im technischen Verfahren eingesetzt werden. Anzüchtung und Selektion können hier einfach oder mehrfach wiederholt werden.

Im Rahmen des erfindungsgemäßen Verfahrens ist es aber auch möglich, die Folge der Verfahrensstufen der Mutation gemäß Stufe 2 und der anschließenden Mutantentrennung gemäß Stufe 3 einfach oder mehrfach zu wiederholen, sofern eine noch stärkere Einwirkung der mutagenen Agentien auf die defekten Mutantenstämme zur Erzielung letztendlich optimaler Produktionsergebnisse wünschenswert erscheint. Abschließend wird dann in der Regel gemäß Stufe 4 aufgearbeitet.

Zur Bestimmung der gewünschten besonders aktiven Defektblockmutanten kann ein Standardtest zur Ausbeutebestimmung eingesetzt werden, der in Abwesenheit von Inhibitoren unter den folgenden Versuchsbedingungen durchgeführt wird:

Der zu bestimmende Defektblockmutantenstamm wird im 500 ml Erlenmeyerkolben mit 100 ml Nährlösung folgender Zusammensetzung gezüchtet: 0,8% Pepton, 0,9% Hefeextrakt, 0,3% Glukose 0,06% Tween 80 (Polyoxyäthylensorbitanmonooleat), 0,06% Cholesterin oder Sitosterin, $p_H$ 7,2. Die Kultur wird auf der Schüttelmaschine (Schüttelfrequenz 150 UpM) bei 30°C für 62 Stunden vorgezüchtet, anschließend 0,1% BRIJ 35 (Polyoxyäthylenmonolauryläther) und 0,1% der Sterinverbindung zugegeben und für weitere 120 Stunden bebrütet. Nach Abbruch der Kulturen werden Proben genommen und diese extrahiert und dünnschichtchromatographisch analysiert.

Die erfindungsgemäß bevorzugten Defektblockmutanten liefern eine Ausbeute an 17-C-$\alpha$-Propionsäurederivat und dabei insbesondere $\Delta$-4 BNC und/oder $\Delta$-1,4 BNC von wenigstens 15 Gew.-%, vorzugsweise von wenigstens 30 Gew.-% auf Cholesterin oder auf Sitosterin. Auf Cholesterin liegt die Ausbeute insbesondere bei wenigstens 50 Gew.-%, sie kann z.B. im Bereich von 70 bis 80 Gew.-%—jeweils bezogen auf Sterineinsatz—liegen. Auf Sitosterin liegen die Ausbeuten in der Regel etwas niedriger, können aber auch hier z.B. 40 bis 50 Gew.-% erreichen. Für die Inhibitor-freie Arbeitweise werden damit bisher unbekannte Ergebnisse zugänglich.

Verfahren zur Herstellung von 17-C-Steroid-$\alpha$-propionsäureverbindungen und insbesondere zur Herstellung von 3-Oxopregna-4-en-20-carbonsäure und/oder 3-Oxo-pregna-1,4-dien-20-carbonsäure

Die selektive Umwandlung des als Ausgangsmaterial gewählten Steroidsubstrats, z.B. also einer natürlichen Sterinverbindung, kann nach Schaffung und Auswahl der Defektmutante gemäß dem zuvor beschriebenen Verfahren in an sich bekannter Weise erfolgen. So kann also beispielsweise die als Einsatzmaterial gewählte Steroidverbindung der Kultur während der Inkubationsperiode zugesetzt werden oder sie kann dem Nährmedium vor der Inokulierung der Blockmutanten beigegeben werden. Es kann eine Steroidverbindung oder auch eine Mischung von mehreren Steroidverbindungen eingesetzt werden. Vorzugsweise werden die selektiv abzubauenden Steroidverbindung in der Kultur in Mengen von etwa 0,1 bis 100 g/l verwendet. Die optimale Konzentration der zu transformierenden Sterinverbindung in der Züchtungsstufe ist im allgemeinen Stamm-abhängig und kann durch einfache Vorversuche jeweils ermittelt werden. Im allgemeinen liegt die Konzentration der Sterinverbindung im Medium vorzugsweise nicht über 20 g/l und häufig nicht über 15 g/l, jedoch werden Mengen über 1 g/l bevorzugt.

Es kann dabei bevorzugt sein, das dem Seitenkettenabbau zu unterwerfende Substrat nicht auf einmal dem Reaktions-medium zuzusetzen, sondern diese Zugabe nach und nach im Verlauf der Reaktion vorzunehmen. Vorzugsweise wird das Ausgangssubstrate in dieser Ausführungsform im wesentlichen kontinuierlich im Reaktionsgemisch im Verlauf der Abbaureaktion zugegenben. Auf diese Weise kann häufig die Ausbeute der gewünschten Abbauprodukte erhöht werden.

Die Kultur wird in einem Nährmedium gezüchtet, das als Kohlenstoffquelle entweder die zu transformierenden Sterine oder aber auch noch zusätzliche metabolisierbare Kohlenstoffquellen sowie die üblicherweise von diesen Mikroorganismen benötigten Nähr- und Wuchsstoffe enthält. Besonders günstig für das Wachstum der Mikroorganismen sind z.B. Paraffin, Glycerin, Carbonsäure, Stärke, Dextrin, Saccharose, Glucose, Fructose und zuckerhaltige Abfallstoffe. Geeignete Stickstoffquellen sind Ammoniumsalze, Nitrate, Pepton, Maisquellwasser, Sojamehl, Schlempe und Fischmehl. Weiterhin können Fermentationsbeschleuniger, wie Hefeextrakt und Vitamine zugesetzt werden. Das Nährmedium enthält darüberhinaus zweckmäßigerweise anorganische Salze, wie Natrium-, Kalium- oder Ammoniumphosphate sowie Calcium-, Magnesium-, Mangan- oder Eisensalze.

Die Emulgierung der Sterine in dem Nährmedium erfolgt vorzugsweise mittels bekannter Emulgatoren, z.B. mittels Fettsäure-Sorbitanester oder deren Äthylenoxidaddukten, Polyoxyäthylenmonolauryläther oder Fettsäureamidoalkylbetain.

Das verwendete Kulturmedium wird vor Beginn der Bakterienanzucht zweckmäßigerweise durch Erhitzen sterilisiert. Nach dem Abkühlen und Beimpfen des Kulturmediums mit einer geeigneten Vorkultur des transformierenden Bakterienstammes wird zwischen 25 bis 55°C inkubiert, vorzugsweise bei 27 bis 30°C. Der $p_H$-Wert der Nährlösung liegt zwischen $p_H$ 4 bis 8,5, vorzugsweise bei 7,0 bis 8,0. Die Kultur wird durch Schütteln, Rühren oder Gaseinleiten mit Sauerstoff versorgt und bis zum vollständigen Abbau des Sterins bis zur gewünschten Stufe inkubiert. Der Abbau des Sterins fordert je nach Substratkonzentration und den anderen Fermentationsbedingungen in der Regel 24 bis 160 Stunden.

Das auf diese Weise hergestellte Verfahrensprokukt, das sich üblicherweise in der Fermentationsbrühe anreichert, kann dann in an sich bekannter Weise aus dem Reaktionsgemisch gewonnen werden. So können beispielsweise BNC-Verbindungen durch Extraktion mit organischen Lösungsmitteln, wie Methylisobutylketon, Essigsäureester, n-Hexanol, n-Octanol, Chloroform oder n-Hexan, aus dem Kulturmedium vor oder nach Abtrennen der Zellen isoliert werden.

Beispielsweise gelingt eine Isolierung von BNC-Verbindungen, indem man einen Liter einer Fermentationslösung, die 1 g BNC enthält, mit etwa gleichen Volumina organischer Lösungsmittel, wie mit Methylisobutylketon, Essigsäureester, n-Hexanol, n-Octanol, Chloroform oder n-Hexan im Perforator, Turborührer oder Scheidetrichter extrahiert. In den beiden letztgenannten Fällen muß zur Abtrennung der BNC-haltigen organischen Phase aus der Emulsion ein Zentrifugationsschritt angeschlossen werden.

Nach Verdampfen des Lösungsmittels hinterbleibt ein BNC-haltiger Rückstand, der nach Umkristallisation z.B. aus Benzol zu reinem BNC vom Smp. 233 bis 236°C aufgearbeitet werden kann.

Die Lösungsmittelextraktion ist insbesondere im sauren $p_H$-Bereich möglich. Dazu wird beispielsweise mit 50%iger $H_2SO_4$ etwa auf $p_H$ 2 eingestellt und mit Methylisobutylketon, Essigsäureester, n-Hexanol, n-Octanol, Chloroform oder n-Hexan in der zuvor angegebenen Weise extrahiert. Nach Eindampfen der organischen Phase wird auch hier ein BNC-haltiger Rückstand erhalten, der wiederum nach Umkristallisation zum Reinprodukt mit dem Smp. 233 bis 236°C führt. Die Extraktion kann auch im neutralen Bereich durchgeführt werden.

Alternativ ist auch eine Reinigung durch Anionenaustausch möglich. Hierzu wird die Fermentationslösung zweckmäßigerweise zunächst aufkonzentriert und dann auf einen alkalischen Wert, beispielweise $p_H$ 12, eingestellt. Nach Zusatz einer beschränkten Menge Methanol und Verrühren im Turborührer wird die Zellmasse mittels einer Durchlaufzentrifuge abgetrennt. Der wäßrigmethanolische Überstand wird über eine Ionenaustauschersäule gepumpt, die mit einem Anionenaustauscherharz, z.B. in Acetatform, gefüllt ist. Die gebildete BNC wird dabei vollstängig im Ionenaustauscher gebunden. Durch Elution mit 10%iger Essigsäure in Methanol gelangt man zu einem Produkt, das zunächst überwiegend BNC enthält, nach Umkristallisation wird auch hier reine BNC vom Smp. 233 bis 236°C erhalten.

Nach einer bevorzugten Ausführungsform gelingt die Isolierung der BNC-Verbindungen aus der Fermenterflüssigkeit ganz einfach durch Ausfällung im sauren Bereich und Abfiltrieren. Hierzu wird die alkalisch eingestellte Fermenterflüssigkeit zunächst filtriert, um das Zellmaterial und andere feste Bestandteile zu entfernen. Anschließend wird angesäuert, dabei fällt BNC in fester filtrierbarer Form aus und kann z.B. durch einfaches Abnutschen gewonnen werden.

Zum Ansäuern ist jede Mineralsäure verwendbar, es können aber auch organische Säuren, z.B. Essigsäure, und sogar gasförmiges $CO_2$ eingesetzt werden. Bei $p_H$ 5 ist die praktisch vollständige Ausfällung erreicht. Zur besseren Filtrierbarkeit kann es günstig sein, die Suspension kurz zu erhitzen.

Der isolierte Feststoff kann bis zu 90% BNC-Verbindungen enthalten. Die reinen Verbindungen lassen sich durch Umkristallisation daraus gewinnen.

Spezielle Einzelheiten zur Erfindung

Die Lehre der Erfindung ist nicht nur mit natürlichen Steroidsubstraten wie Cholesterin, Sitosterin usw. durchführbar. Geeignete Ausgangsmaterialien für eine Transformation zu beispielsweise Δ-4- und/oder Δ-1,4 BNC sind auch Abkömmlinge hiervon, wie Cholestenon, Sitostenon, Stigmastenon und dergleichen. Im Rahmen der Entwicklungsarbeiten wurde gefunden, daß ein zu der Gruppe der coryneformen Bakterien gehörender Wildstamm ein besonders geeignetes Ausgangsmaterial zur Gewinnung der gewünschten Defektblockmutanten sein kann. Dieser Stamm, der unter der Hinterlegungsnummer CBS 660.77 in Baarn, Niederlande hinterlegt ist, wurde morphologisch und wachstumsphysiologisch näher untersucht. Es wurden dabei folgende Eigenschaften festgestellt:

| | |
|---|---|
| 1. Zellform | unregelmäßig, überwiegend kurze Stäbchen, teilweise kokkoid oder auch längere Stäbchen, teilweise verzweigt, zum Teil gewinkelt; sehr junge Kulturen: mit überwiegend langen, schwach verzweigten Stäbchen. |
| 2. Sporenbildung | negativ |
| 3. Gramfärbung | positiv |
| 4. Kolonienform auf Bouillonagarplatten | kleine, runde, cremefarbige Kolonien, glatter Rand, konvex, glatt, glänzend. |
| 5. Wachstum auf Glucose-Agar | Wachstum etwas üppiger, sonst wie unter 4. |
| 6. Wachstum auf Glucose-Nitrat-Agar | Wachstum etwas üppiger, sonst wie unter 4. |
| 7. Wachstum auf Kartoffelagar | Kolonien im Verband etwas rötlich, sonst wie unter 4. |
| 8. Wachstum auf Glucose-Asparagin-Agar | Kolonien sehr viel kleiner, rund, cremefarbig, glatter Rand, glänzend. |
| 9. Wachstum auf Stärke-Agar | mittelgroße, runde Kolonien, z.T. gelappt, cremefarbig, glänzend, feuchter als auf BA. |
| 10. Wachstum auf Calcium-Caseinat-Agar | langsames Wachstum, keine caseolytische Hofbildung. |
| 11. Wachstum auf Magermilch | negativ und keine Koagulation. |
| 12. Indolbildung aus Tryptophan | negativ. |
| 13. Säurebildung/ Methylrotprobe | negativ. |
| 14. Voges-Proskauer-Reaktion (Acetoinbildung): | negativ. |
| 15. Wachstum auf Citrat | sehr schwach bis negativ. |
| 16. Reduktion von $NO_3$ zu $NO_2$ | negativ. |
| 17. Reduktion von $NO_2$ | schwach positiv. |
| 18. Kohlehydratverwertung | Wachstum auf Glucose, Fructose, Saccharose, Maltose, Mannose, Lactose, Galactose, Mannit, Rhamnose, Arabinose und Stärke; jedoch in keinem Fall Gas- oder Säurebildung. |

| | |
|---|---|
| 19. NaCl-Brühe | gutes Wachstum bis 3% NaCl, schwaches Wachstum bis 5%, kein Wachstum ab 7%. |
| 20. Harnstoffspaltung | positiv. |
| 21. Sauerstoffbedarf | strikt aerob. |
| 22. Wachstums-Temperaturen | Wachstum zwischen 19 u. 37°C, gutes Wachstum um 30°C. |
| 23. Wachstum auf | kleine weiße Kolonien nach 6 Tagen. |

0,5 g $NaH_2PO_4$

1,8 g $K_2HPO_4$

0,5 g $MgSO_4 \times 7H_2O$

0,2 g $MnSO_4 \times 4H_2O$

0,2 g $CaCl_2 \times 2H_2O$

0,02 g $FeSO_4 \times 7H_2O$

0,5 g $NH_4NO_3$

1,0 g Tween 80

2,0 g Cholesterin

1000 ml dest. $H_2O$, $p_H$ 6,8

Dieser Wildstamm wird durch Behandlung mit 1-Methyl-3-nitro-1-nitrosoguanidin wie in den folgenden Beispielen beschrieben mutiert. Die Mutantenpopulation wird im erfindungsgemäßen Verfahren aufgearbeitet. Es wurde dabei die neue Blockmutante gewonnen, die in Baarn, Niederlande, unter der Hinterlegungsnummer CBS 437.77 hinterlegt ist. Diese neue Blockmutante ist zur inhibitorfreien Herstellung von 3-Oxo-pregna-4-en-20-carbonsäure und/oder 3-Oxo-pregna-1,4-dien-20-carbonsäure auch in Abwesenheit von den Steroidringabbau und/oder das Wachstum hemmenden Inhibitoren geeignet. So wird beispielsweise aus Cholesterin als Ausgangsmaterial Δ-4 BNC und/oder Δ-1,4 BNC in hohen Ausbeuten — neben nur geringen Mengen an AD und/oder ADD — gewonnen.

Beispiel 1

A *Gewinnung geeigneter Wildstämme*

Nach an sich üblichen Anreicherungsmethoden werden aus Erdproben sterinabbauende Mikroorganismen isoliert, die man zunächst in einem Agarplattentest auf ihre Fähigkeit untersucht, auf Cholesterin zu wachsen. Stämme, die mit Cholesterin als einziger C-Quelle deutliches Koloniewachstum zeigen, werden weiteren Selektionsverfahren unterworfen.

B *Bestimmung des Wachstumsfaktors a*

Zur Ermittlung des Wachstumsfaktors a werden die isolierten Reinkulturen aerob in Schüttelkulturen in folgendem Medium angezüchtet (pro Liter):

1,0 g $KH_2PO_4$

0,5 g $MgSO_4 \cdot 7\,H_2O$

0,1 g NaCl

0,1 g $CaCl_2 \cdot 2\,H_2O$

5,0 g $(NH_4)_2SO_4$

| | |
|---|---|
| 1,0 g | Tween 80 (Polyoxyäthylensorbitanmonooleat) |
| 2,0 g | 17-C-Seitenkettensterinverbindung, z.B. Cholesterin |
| 0,5 g | Hefeextrakt |
| 0,01 g | 1-Histidin |
| 0,02 g | dl-Methionin |
| 0,02 g | dl-Tryptophan |
| 2 $\mu$g | Biotin |
| 400 $\mu$g | Calciumpantothenat |
| 2 $\mu$g | Folsäure |
| 2000 $\mu$g | Inosit |
| 400 $\mu$g | Niacin |
| 200 $\mu$g | p-Aminobenzoesäure |
| 400 $\mu$g | Pyridoxinhydrochlorid |
| 200 $\mu$g | Riboflavin |
| 400 $\mu$g | Thiaminhydrochlorid |

Spurenelementlösung

$p_H$-Wert des Nährmediums 6,8.

Zur Bestimmung der optischen Dichte der Zellsuspension werden jeweils in 24-stündigen Abständen Proben genommen und die Extinktion (E') im Zeiss-Photometer (Typ PL 4) bei einer Wellenlänge von 620 nm (d = 1 cm) gegen destilliertes Wasser gemessen. Der Wachstumsfaktor a ergibt sich als maximale optische Dichte am Ende der logarithmischen Wachstumsphase minus der Ausgangsextinktion $E_0$.

C *Bestimmung des Selektionsfaktors b*

Diejenigen Wildstämme, die im Schüttelkolbentest mit Cholesterin das beste Wachstum zeigen, werden anschließend im Warburgtest mit 26-$^{14}$C- und 4-$^{14}$C-markiertem Cholesterin auf ihre Fähigkeiten untersucht, die Seitenkette des Cholesterins abzubauen.

Die Anzucht der Stämme erfolgt in 500 ml Erlenmeyerkolben mit 150 ml Nährlösung folgender Zusammensetzung: 1,56% Pepton, 0,28% Hefeextrakt, 0,56% NaCl, 0,10% Glukose, 0,10% Tween 80, 0,05% Cholesterin, $p_H$ 7,2. Die Stämme werden 48 Stunden bei 30°C auf der Schüttelmaschine angezüchtet, die Zellen anschließend abzentrifugiert, 2 mal 0,05 m $PO_4$-Puffer ($p_H$ 7,2) gewaschen und im gleichen Volumen Puffer resuspendiert.

Für den Warburg-Versuch werden 2,4 ml Zellsuspension mit 0,2 ml Cholesterinlösung (5 u Mol Cholesterin und 20000 cpm 4-$^{14}$C oder 26-$C^{14}$) bebrütet. Das abgespaltene, radioaktive $CO_2$ wird in 0,2 ml Äthanolamin (im Zentralgefäß) aufgefangen. Die Reaktion wird nach 6 Stunden bei 30°C durch Zukippen von 0,2 ml 6 n $H_2SO_4$ gestoppt und gleichzeitig das noch in der Suspension vorhandene $CO_2$ ausgetrieben. Zur Messung des absorbierten $^{14}CO_2$ werden dem Warburggefäß 0,1 ml Äthanolamin entnommen und die Aktivität im Sczintillation-Counter (Fa. Berthold u. Frieseke, Typ bF 5 000) gemessen. Der Selektivitätsfaktor b ist die Quotient aus 26-$^{14}CO_2$/4-$^{14}CO_2$.

Gemäß dieser Vorgehensweise wurde schließlich ein Wildstamm selektioniert, der einen Wachstumsfaktor a = 3,71 aufwies und einen Selektivitätsfaktor b = 5,0. Dieser Stamm wurde zur Gewinnung der gewünschten Blockmutanten herangezogen.

Im folgenden wird eine Reihe von Wildstämmen gezeigt, die im Warburgtest einen günstigen Selektivitätsfaktor b(26-$^{14}CO_2$/4-$^{14}CO_2$) aufweisen und von der Anmelderin isoliert worden sind:

| Interne Bezeichnung | Hinterlegungs-Nummer | Selektions-faktor b |
|---|---|---|
| SC— 18 | DSM 1 419 | 1,1 |
| SC— 89 | DSM 1 421 | 1,2 |
| SC—338 | DSM 1 425 | 1,7 |
| SC—358 | DSM 1 427 | 1,2 |
| SC—372 | DSM 1 428 | 2,3 |

Beispiel 2

A *Mutation*

Ausgehend vom Wildstamm des Beispiels 1,C wurde nach der bereits ausführlich beschriebenen Mutations- und Selektionsmethode die Mutante CBS 437.77 isoliert.

B *Gewinnung von Mutanten mit gesteigerten BNC-Ausbeuten*

1. Mutationsbehandlung mittels UV-Bestrahlung:

Zur Steigerung der BNC-Ausbeuten wird dieser Mutantenstamm einer erneuten Mutationsbehandlung unterzogen. Der Stamm wird zunächst im folgender Nährlösung bei 30°C auf der Schüttelmaschine vermehrt (Nährlösung A): 0,8% Pepton, 0,9% Hefeextrakt, 0,3% Glukose, 0,06% Tween 80, 0,06% Cholesterin, $p_H$ 7,2.

Nach Erreichen der logarithmischen Wachstumsphase werden die Zellen abzentrifugiert, 2 mal mit sterilem 0,1 m $PO_4$-Puffer ($p_H$ 6,5) gewaschen, im gleichen Puffer resuspendiert und unterm Mikroskop die Zelldichte auf $10^8$ Zellen/ml eingestellt. 8 ml dieser Zellsuspension werden in eine Petrischale überführt und 90 Sekunden unter der UV-Lampe bestrahlt (Abstand 30 m, UV-Bestrahlungslampe der Fa. Schütt Jun., Göttingen).

C *Selektion und Isolierung der gewünschten Mutantenstämme*

1. Anreicherung von Stämmen mit verminderter Endprodukt-Hemmung:

Die UV-bestrahlte Zellsuspension wird anschließend in folgender Nährlösung angezüchtet: 0,05% $NaH_2PO_4$, 0,20% $K_2HPO_4$, 0,05% $MgSO_4 \cdot 7H_2O$, 0,02% $CaCL_2 \cdot 2H_2O$, 0,005% $MnSO_4 \cdot 4H_2O$, 0,005% $(Fe)_2SO_4 \cdot 7H_2O$, 0,10% $(NH_4)_2SO_4$, 0,0001% Biotin, 0,10% BRIJ 35 (Polyoxyäthylenmonolauryläther), 0,50% Cholesterin, 0,50% BNC, $p_H$ 7,2. Nach 72-stündiger Inkubationsdauer bei 30°C wird 1 ml Kulturlösung in frische Nährlösung A überführt und erneut 48 Stunden bei 30°C aerob kultiviert.

2. Penicillin-Methode:

Zur Eliminierung möglicher Revertanten wird die angewachsene Kulturlösung anschließend einer Penicillinbehandlung unterworfen. 0,1 ml dieser Zellsuspension werden in 10 ml folgender Nährlösung übertragen: 0,05% $NaH_2PO_4$, 0,20% $K_2HPO_4$, 0,05% $MgSO_4 \cdot 7H_2O$, 0,02% $CaCl_2 \cdot 2H_2O$, 0,005% $MnSO_4 \cdot 4H_2O$, 0,005% $(Fe)_2SO_4 \cdot 7H_2O$, 0,10% $(NH_4)_2SO_4$, 0,0001% Biotin, 0,10% BRIJ 35 (Polyoxyäthylenmonolauryläther), 0,10% BNC, $p_H$ 7,2. Nach 18-stündigem Schütteln bei 30°C wird mit frischer, vorgewärmter Nährlösung derselben Zusammensetzung auf ca. $10^6$ Zellen pro ml verdünnt, 1000 IU Penicillin G zugesetzt und weitere 5 Stunden bei 30°C inkubiert. Dann wird das Antibiotikum durch Abzentrifugieren der Zellen und Waschen mit steriler, physiologischer Kochsalzlösung entfernt und die Zellen in obengenannten Medium mit 1,0% Cholesterin anstelle von BNC für weitere 48 Stunden inkubiert. Anschließend wird die Zellsuspension auf demselben Nährmedium plus 1,6% Agar ausplattiert und die Kolonien, die auf diesem Medium mit erhöhter Substratkonzentration das beste Wachstum zeigen, als Stammkulturen angelegt.

Auf diese Weise wurden Stämme isoliert, die nach Inkubation mit Cholesterin gute Ausbeuten an BNC erbrachten. Folgende dieser Stämme wurden bei der Deutschen Sammlung für Mikroorganismen (DSM) in Göttingen hinterlegt.

| Interne Bezeichnung | Hinterlegungs-Nummer | BNC-Ausbeuten |
|---|---|---|
| T 137 | DSM 1 435 | 315 mg/100 ml |
| T 139 | DSM 1 437 | 305 mg/100 ml |
| T 162 | DSM 1 439 | 321 mg/100 ml |
| T 166 | DSM 1 442 | 308 mg/100 ml |
| T 190 | DSM 1 443 | 318 mg/100 ml |
| T 191 | DSM 1 444 | 392 mg/100 ml |
| T 244 | DSM 1 445 | 302 mg/100 ml |

## Beispiel 3

A *BNC-Ausbeuten der aktivsten Mutantenstämme*

Die Stämme wurden in 500 ml Erlenmeyerkolben mit 100 ml Nährlösung folgender Zusammensetzung aerob gezüchtet: 0,5% Pepton, 0,8% Hefeextrakt, 0,4 % Maiskleber, 0,3% Glukose, 0,05% Tween 80, 0,05% Cholesterin, $p_H$ 7,2. Die Kultur wurde auf der Schüttelmaschine (Schüttelfrequenz 150 UpM) bei 30°C für 48 Stunden vorgezüchtet, anschließend 0,2% Emulgator und 0,5% Cholesterin zugegeben und für weitere 120 Stunden bebrütet. Nach Abbruch der Kulturen wurden Proben genommen, auf $p_H$ 2,0 eingestellt, 1:1 mit Essigester extrahiert und dünnschichtchromatographisch analysiert. Die Ausbeuten der einzelnen Stämme sind im Beispiel 2 in der Tabelle unter C/2 angegeben.

## Beispiel 4

Die Akkumulation der unerwünschten Metabolite Cholest-4-en-3-on und Cholesta-1,4-dien-3-on aus dem Substrat Cholesterin, durch den Stamm CBS 437.77 kann weitgehend unterdrückt werden, wenn das zu transformierende Substrat kontinuierlich dem Reaktionsansatz zugesetzt wird. Entsprechendes gilt für die anderen Sterinsubstrate.

Zwei 1 l-Fermenter mit 400 ml steriler Nährlösung (0,9% Pepton, 0,9% Maisquellwasser, 0,9% Hefeextrakt, 0,5% $K_2HPO_4$, 0,5% Glycerin, 0,1% Polypropylenglykol, $p_H$ 6,5) werden mit 50 ml einer gut angewachsenen Vorkultur des Stammes CBS 437.77 beimpft, die in einem Nährmedium (0,9% Pepton, 0,9% Hefeextrakt, 0,5% Glycerin, $p_H$ 6,5) 48 Stunden bei 30°C inkubiert worden war. Die Fermenter werden bei 30°C gerührt und mit 500 ml Luft/Minute belüftet.

In einen Fermenter wird nach 24 Stunden Inkubation eine Emulsion von 5 g Cholesterin, 1 g Sorbitanmonostearat, 4 ml Polypropylenglykol und 4 ml 1 N NaOH in 100 ml $H_2O$ über eine Dosierpumpe in den Fermenter gegeben. Die Dosierpumpe förderte pro Stunde 5 ml Suspension, so daß sich die Substratzugabe von der 24. Fermentationsstunde bis in die 44. Fermentationsstunde erstreckte. Nach 44 und 78 Stunden wurden aus dem Fermenter 100 ml Kulturbrühe entnommen, nach Ansäuern mit Schwefelsäure und Extraktion mit Methylisobutylketon wurden die Fermentationsprodukte quantitativ mittels Dünnschichtchromatographie bestimmt.

In dem Kontrollansatz wurde die gesamte Substratmenge nach 24 Stunden in dem Fermenter gegeben.

Bei kontinuierlicher Zugabe des Substrates wurden nach 44 Stunden Fermentationsdauer folgende Menge bestimmt (mg/100 ml): Cholesterin 120, Cholestenon plus Cholestadienon 40, Bisnorcholenonsäure 100; nach 78 Stunden Fermentation: Cholesterin 20, Cholesteron plus Cholestadienon 5, Bisnorcholenonsäure 260.

Bei absatzweiser Zugabe des Substrates wurden nach 44 Stunden Fermentationsdauer bestimmt (mg/100 ml): Cholesterin 100, Cholestenon plus Cholestadienon 240, Bisnorcholenonsäure 80; nach 78 Stunden Fermentation: Cholesterin 40, Cholestenon 100, Bisnorcholenonsäure 180.

## Beispiel 5

Gewinnung der Mutante CBS 437.77 vom Wildstamm CBS 660.77.

A) Zahlreiche sterinabbauende Mikroorganismen waren nach den üblichen Anreicherungsmethoden aus Erdproben isoliert worden. Unter diesen Stämmen wurde anschließend durch Inkubation mit 4—14 C und 26—14 C markiertem Cholesterin nach Mikroorganismen gesucht, die Sterine bevorzugt von der Seitenkette her abbauen. Dabei zeigte der Stamm CBS 660.77 mit 26—14 C-Cholesterin die höchsten Abbauraten und erwies sich damit zur Selektion abbaudefekter Mutanten als besonders geeignet.

Die nachfolgend beschriebene Methode der Mutantenisolierung ist nicht nur anwendbar auf den

vorliegenden, zur Gruppe der coryneformen Bakterien gehörenden Wildstamm, sondern auch auf jede andere Spezies.

Die Mutation des Wildstammes erfolgte durch Behandlung mit 1-Methyl-3-nitro-1-nitrosoguanidin. Die Zellen wurden 48 Stunden bei 30°C in Medium A angezüchtet.

| | |
|---|---|
| Medium A: | 1,0% Hefeextrakt |
| | 1,0% Pepton |
| | 0,5% Glucose |
| | $p_H$ 7,5. |

Die Zellkultur mit einem Zelltiter von 2 × $10^8$ Zellen/ml wurde 1 Stunde bei 30°C mit der mutagenen Verbindung behandelt (1 mg/ml), anschließend gewaschen und weitere 3 Tage in Nährmedium B gezüchtet bis zu einem Zelltiter von $<10^9$ Zellen/ml.

| | |
|---|---|
| Medium B: | 0,05% Natriumdihydrogenphosphat |
| | 0,20% Dikaliumhydrogenphosphat |
| | 0,05% Magnesiumsulfat × 7 $H_2O$ |
| | 0,02% Calciumchlorid × 2 $H_2O$ |
| | 0,005% Mangansulfat × 4 $H_2O$ |
| | 0,005% Eisen-II-sulfat × 7 $H_2O$ |
| | 0,10 % Ammoniumsulfat |
| | 0,0001% D-Biotin. |

B) 0,1 ml dieser so erhaltenen Kulturlösung wurden in 10 ml Nährmedium B mit Zusatz von 0,1% Polyoxymethylenmonolauryläther un 0,1% 3-Oxo-pregna-1,4-dien-20-carbonsäure übertragen und 36 Stunden bei 30°C inkubiert. Zur Förderung der Mutanten mit Defekten im Ringabbausystem wurde mit frischem, vorgewärmten Nährmedium derselben Zusammensetzung auf $10^6$ Zellen/ml verdünnt, 1000 IU Penicillin G/ml zugesetzt und weitere 24 Stunden bei 30°C inkubiert. Dann wurden die Zellen gewaschen und auf Agarplatten mit Nährmedium B plus 2% Agar und 0,5% Glycerin ausplattiert. Nach Vermehrung der Zellen zu sichtbaren großen Kolonien wurde mit der Replica-Stempeltechnik auf Nähragar B plus 0,1% Polyoxyäthylenmonolauryläther und 0,1% 3-Oxo-pregna-1,4-dien-20-carbonsäure abgestempelt und erneut inkubiert. Im Rahmen dieses Screenings wurden mehr als 50 Mutantenkolonien isoliert, die nach dem Abstempeln auf dem steroidhaltigen Nährboden nicht mehr anwuchsen.

Zur Auswahl der interessantesten Mutanten wurden die Stämme in 2 ml Nährlösung A eingeimpft, 24 Stunden bei 30°C inkubiert, danach zusätzlich mit 0,2% Polyoxyäthylenmonolaurylähter und 0,2% Cholesterin versetzt und nach weiteren 72 Stunden die Kulturlösung analysiert. Der Inhalt der Röhrchen wurde jeweils mit 0,5 ml Methylisobutylketon ausgeschüttelt, zentrifugiert, aliquote Mengen der organischen Phase auf Merk-Silicagelplatten aufgetragen und in dem Laufmittelgemisch Benzol-Äthylacetat (70 + 30) entwikkelt. Der Nachweis der Steroide auf der Chromatographieplatte wurde durch Ansprühen mit dem Gemisch konz. $H_2SO_4$ + Äthanol (1 + 1) und anschließendes Erhitzen auf 120°C durchgeführt. Hierbei wurd der Stamm CBS 437.77 als Mutante erkannt, die einen Block im Abbau des Sterinringsystems besitzt.

Beispiel 6

Zur genaueren Prüfung der Sterin-Transformation durch Corynebacterium CBS 437.77 wurde der Stamm in einem Schüttelkolbenansatz mit 100 ml Nährlösung A bei 30°C und einer Schüttelfrequenz von 150 UpM für 24 Stunden vorgezüchtet. Danach wurden 0,1% Cholesterinlösung zugegeben und zu verschiedenen Zeiten Proben genommen und analysiert.

20 ml der Kulturlösung wurden mit 2 n-Schwefelsäure auf $P_H$ 2,0 eingestellt und im Perforator 3 Stunden mit Methylisobutylketon, Essigsäureester, n-Hexanol, n-Octanol, Chloroform oder n-Hexan extrahiert. Aliquote Mengen der organischen Phase wurden auf Merck-Silicagelplatten aufgetragen und in dem Laufmittelgemisch Toluol-Butanon (80 + 20) entwickelt. Die Auswertung der Chromatographie

erfolgte mit einem Dünnschichtscanner bei 250 nm. Als Hauptkomponente der Cholesterintransformation wurden gefunden 50 mg 3-Oxo-Pregna-1,4-dien-20-carbonsäure neben geringen Mengen Androst-4-en-3,17-dion und Androst-1,4-dien-3,17-dion.

Beispiel 7

Zur weiteren Prüfung der Sterin-Transformation durch Corynebacterium spec. CBS 437.77 wurde der Stamm in einem Schüttelkolbenansatz mit 100 ml Nährlösung A bei 30°C und einer Schüttelfrequenz von 150 UpM für 24 Stunden vorgezüchtet. Danach wurden 0,1% Polyoxyäthylensorbitanmonooleat und 0,1% Sitosterinlösung zugegeben und zu verschiedenen Zeiten Proben genommen und analysiert.

20 ml Kulturlösung wurden mit 2 n-Schwefelsäure auf $p_H$ 2,0 eingestellt und im Perforator 3 Stunden mit Essigsäureäthylester extrahiert. Aliquote Mengen der organischen Phase wurden auf Merck-Silicagelplatten aufgetragen und in dem Laufmittelgemisch Toluol-Butanon (80 + 20) entwickelt. Die Auswertung der Chromatographie erfolgte mit einem Dünnschichtscanner bei 250 nm. Dabei wurde die Bildung von 20-Carboxypregna-1,4-dien-3-on und 20-Carboxy-pregna-4-en-3-on neben geringen Mengen Androst-4-en-3,17-dion und Androst-1,4-dien-3,17-dion nachgewiesen.

Beispiel 8

Die Kulturlösung aus Beispiel 6 wurde im Rotationsverdampfer auf 1/4 ihres Ausgangsvolumens eingeengt, mit NaOH auf $p_H$ 13 eingestellt, mit dem dreifachen Volumen Methanol versetzt und die Zellmasse anschließend abzentrifugiert. Die zellfreie Kulturlösung wurde über eine Anionenaustauschersäule in Acetatform gegeben und durch Elution mit Methanol zunächst Androst-4-en-3,17-dion, Androst-1,4-dien-3,17-dion und Spuren von Cholestenon abgetrennt. 20-Carboxy-pregna-4-en-3-on und 20-Carboxy-pregna-1,4-dien-3-on wurden nachfolgend mit 10%iger Essigsäure vom Ionenaustauscher getrennt und angereichert. Das angereicherte Eluat wurde bis zur Trockene eingedampft, mit 5%igem Ammoniak unter leichtem Erwärmen aufgenommen, auf 4°C abgekühlt und die ausgefallenen Produkte abfiltriert. Durch wiederholte Solubilisierung, Extraktion in Äther und Umkristallisierung aus Benzol ließ sich das 3-Oxo-pregna-1,4-dien-20-carbonsäure weitestgehend reinigen.

**Patentansprüche**

1. Verfahren zur Herstellung von Mikroorganismen-Defektblockmutanten, die zur technischen Gewinnung von 17-C-Steroid-$\alpha$-propionsäureverbindungen, insbesondere zur Herstellung von 3-Oxo-pregna-4-en-20-carbonsäure ($\Delta$-4 BNC) und/oder 3-Oxo-pregna-1,4-dien-20-carbonsäure ($\Delta$-1,4 BNC), aus 17-C-Seitenketten-Steroidsubstraten, z.B. aus Sterinverbindungen tierischen oder pflanzlichen Ursprungs, durch wenigstens weitgehend selektiven Seitenkettenabbau auch in Abwesenheit von den Steroidringabbau und/oder das Wachstum hemmenden Inhibitoren unter aeroben Bedingungen befähigt sind, dadurch gekennzeichnet, daß man

1) einen Mikroorganismen-Wildstamm isoliert und züchtet, der zum aeroben Wachstum auf Sterinverbindungen als alleiniger Kohlenstoffquelle befähigt ist und dabei den 17-C-Seitenkettenabbau einem Ringabbau genenüber bevorzugt,
2) den Wildstamm einer an sich bekannten Mutationsbehandlung unterwirft,
3) die Mutantenpopulation auf einem Trennmedium züchtet, auf dem die 17-C-Steroid-$\alpha$-propionsäureverbindungen liefernden Mutanten nicht oder praktisch nicht wachsen, während die unerwünschten begleitenden Mutantenstämme wachsen und hierdurch bzw. während ihres Wachstums abgetötet werden und
4) den verbliebenen Rest der Mutantenstämme auf 17-C-Seitenkettensterin-Verbindungen züchtet und dabei die Stämme mit optimaler Produktion der 17-C-Steroid-$\alpha$-propionsäureverbindungen isoliert.

2. Verfahren nach Anspruch 1, dadurch gekennzeichnet, daß die Verfahrensstufen der Mutation 2) und der Mutantentrennung 3) eine- oder mehrfach an den noch lebensfähigen Mutantenstämmen aus der Mutantentrennung 3) wiederholt werden.

3. Verfahren nach Ansprüchen 1 und 2, dadurch gekennzeichnet, daß Wildstämme ausgewählt und gezüchtet werden, die beim aeroben Wachstum auf Sterinverbindungen mit gesättigten oder ungesättigten Alkylresten in 17-C- mit 8 bis 10 C-Atomen eine selektive Abbauleistung unter Standardbedingungen gemäß der allgemeinen Formel

$$I = a \cdot 10^b$$

ergeben, worin a der Wachstumsfaktor und b der Selektivitätsfaktor des Wildstammwachstums sind und der Selektivitätsindex I den Zahlenwert von wenigstens 10, vorzugsweise von wenigstens 100 und insbesondere den Wert von wenigstens $10^5$ beträgt.

4. Verfahren nach Ansprüchen 1 bis 3, dadurch gekennzeichnet, daß Wildstämme ausgewählt und eingesetzt werden, deren Selektivitätsfaktor b wenigstens 1, vorzugsweise wenigstens 2, und

deren Wachstumsfaktor a vorzungsweise wenigstens 0,2, insbesondere ebenfalls wenigstens 1 beträgt, wobei es weiterhin bevorzugt sein kann, die Wildstämme zunächst nach ihrem Selektivitätsfaktor b—bei möglichst hohen Zahlenwerten für b—und erst dann nach ihrem Wachstumsfaktor a zu berücksichtigen.

5. Verfahren nach Ansprüchen 1 bis 4, dadurch gekennzeichnet, daß die Züchtung der Wildstämme unter aeroben Bedingungen in Gegenwart eines wäßrigen Nährmediums auf Sterinen pflanzlichen oder tierischen Ursprungs, beispielsweise auf Cholesterin, Sitosterin, Stigmasterin und/oder Campesterin erfolgt, wobei vorzugsweise die ausgewählte Sterinverbindung als einzige Kohlenstoffquelle zur Züchtung der Wildstämme eingesetzt wird.

6. Verfahren nach Anspruch 5, dadurch gekennzeichnet, daß schon die Züchtung der Wildstämme auf der Sterinverbindung als Kohlenstoffquelle erfolgt, die mittels der schließlich erhaltenen Defektblockmutanten zur 17-C-Steroiod-$\alpha$-propionsäureverbindung abgebaut werden soll.

7. Verfahren nach Ansprüchen 1 bis 6, dadurch gekennzeichnet, daß die Mutation der ausgewählten Wildstämme mit energiereicher Strahlung oder durch Behandlung mit mutagenen Agentien, z.B. durch Behandlung mit Nitrosoguanidinen und/oder Äthylmethansulfonat, durchgeführt wird.

8. Verfahren nach Ansprüchen 1 bis 7, dadurch gekennzeichnet, daß als Trennmedium für eine bei der Mutation angefallene Mutantenpopulation ein Wäßriges Nährmedium eingesetzt wird, das als Kohlenstoffquelle eine Steroidverbindung mit einer 17-C-Seitenkette mit bis zu 5 C-Atomen oder auch in 17-C keine Seitenkette enthält, wobei jedoch vorzugsweise eine Steroidverbindung mit 3 C-Atomen in der 17-C-Seitenkette verwendet wird.

9. Verfahren nach Ansprüchen 1 bis 8, dadurch gekennzeichnet, daß man als Kohlenstoffquelle im Mutantentrennmedium wenigstens eine 17-C-Steroid-$\alpha$-propionsäureverbindung einsetzt, die dabei vorzugsweise als einzige Kohlenstoffquelle oder wenigstens doch als mengenmäßig überwiegende Kohlenstoffquelle zum Einsatz kommt.

10. Verfahren nach Ansprüchen 1 bis 9, dadurch gekennzeichnet, daß im Mutantentrennmedium als Kohlenstoffquelle diejenigen 17-C-Steroid-$\alpha$-propionsäureverbindungen eingesetzt werden, deren Herstellung mittels der erfindungsgemäß gezüchteten Defektblockmutanten letztendlich angestrebt wird.

11. Verfahren nach Ansprüchen 1 bis 10, dadurch gekennzeichnet, daß man die auf dem Mutantentrennmedium wachsenden Stämme in Gegenwart der nicht oder praktisch nicht wachsenden Defektblockmutanten und dabei ohne deren Schädigung abtötet.

12. Verfahren nach Ansprüchen 1 bis 11, dadurch gekennzeichnet, daß man die Abtötung der im Mutantentrennmedium wachsenden Stämme durch Antibiotikazusatz, beispielsweise durch Zusatz einer Penicillinverbindung oder durch Mitverwendung von $P^{32}$-Verbindungen, vornimmt.

13. Verfahren nach Ansprüchen 1 bis 12, dadurch gekennzeichnet, daß man als wenigstens überwiegende Kohlenstoffquelle im Mutantentrennmedium 3-Oxo-pregna-4-en-20-carbonsäure und/oder 3-Oxopregna-1,4-dien-20-carbonsäure einsetzt.

14. Verfahren nach Ansprüchen 1 bis 13, dadurch gekennzeichnet, daß man aus dem Mutantentrennmedium die noch lebensfähigen Mutantenstämme isoliert und daraus die Mutantenstämme mit optimaler Produktivität des gewünschten Verfahrensproduktes gewinnt.

15. Verfahren nach Ansprüchen 1 bis 14, dadurch gekennzeichnet, daß man mit Wildstämmen von Achromobacter, Arthrobacter, Bacillus, Brevibacterium, Corynebacterium, Flavobacterium, Microbacterium, Mycobacterium, Nocardia, Protaminobacterium, Serratia oder Streptomyces arbeitet.

16. Verfahren nach Ansprüchen 1 bis 15, dadurch gekennzeichnet, daß man den Wildstamm Corynebacterium CBS 660.77 einsetzt.

17. Neue Defektblockmutantenstämme CBS 437.77 sowie DSM 1435, 1437, 1439, 1442, 1443, 1444, 1445, die zur inhibitorfreien Herstellung von 3-Oxo-pregna-4-en-20-carbonsäure und/oder 3-Oxo-pregna-1,4-dien-20-carbonsäure geeignet sind.

18. Verfahren zur Herstellung von 3-Oxo-pregna-4-en-20-carbonsäure ($\Delta$-4 BNC) und/oder 3-Oxo-pregna-1,4-dien-20-carbonsäure ($\Delta$-1,4 BNC) durch mikrobiellen Seitenkettenabbau an 17-C-Seitenketten-Steroidsubstraten, dadurch gekennzeichnet, daß man

a) einen auch in Abwesenheit von Inhibitoren zur Hemmung des Steroidringabbaus und/oder des Wachstums $\Delta$-4 BNC und/oder $\Delta$-1,4 BNC in einer Ausbeute von wenigstens 15 Gew.-%—bezogen auf eingesetztes Steroidsubstrat—liefernden Mikroorganismus in einem wäßrigen Nährmedium unter aeroben Bedingungen und in Gegenwart eines 17-C-Seitenketten-Steroidsubstrats unter Anreicherung von 3-Oxo-pregna-4-en-20-carbonsäure und/oder 3 Oxo-pregna-1,4-dien-20-carbonsäure züchtet und

b) anschließend die gebildete 3-Oxo-pregna-4-en-20-carbonsäure und/oder 3-Oxo-1,4-dien-20-carbonsäure isoliert.

19. Verfahren nach Anspruch 18, dadurch gekennzeichnet, daß mit Defektblockmutanten als Mikroorganismen gearbeitet wird, die durch Mutation und anschließende Selektion aus solchen zuvor ausgewählten Wildstämmen gezüchtet worden sind, die auf Steroidverbindungen mit 17-C-Seitenketten als vorzugsweise einziger Kohlenstoffquelle mit wenigstens etwa gleicher Abbaugeschwindigkeit für die Seitenketten wie für den Ringanteil der Steroidverbindungen zu wachsen vermögen, vorzugsweise aber eine erhöhte Seitenkettenabbaugeschwindigkeit besitzen.

20. Verfahren nach Ansprüchen 18 und 19, dadurch gekennzeichnet, daß die eingesetzten

Defektblockmutanten aus Wildstämmen gezüchtet worden sind, die beim Wachstum auf Steroidverbindungen eine selektive Abbauleistung unter Standardbedingungen gemäß der Allgemeinen Formel

$$I = a \cdot 10^b$$

ergeben, worin a der Wachstumsfaktor und b der Selektivitätsfaktor des Wildstamm-Wachstums sind und der Selektivitätsindex I den Zahlenwert von wenigstens 10, vorzugsweise von wenigstens 100 aufweist.

21. Verfahren nach Anspruch 20, dadurch gekennzeichnet, daß der Selektivitätsindex I des Wildstamms wenigstens $10^5$ beträgt.

22. Verfahren nach Ansprüchen 18 bis 21, dadurch gekennzeichnet, daß der Selektivitätsfaktor b des zur Züchtung des Defektblockmutanten eingesetzten Wildstamms wenigstens 2, und der Wachstumsfaktor a vorzugsweise wenigstens 0,2, insbesondere ebenfalls wenigstens 1 beträgt.

23. Verfahren nach Ansprüchen 18 bis 22, dadurch gekennzeichnet, daß die Züchtung der Wildstämme zur anschließenden Selektion gemäß ihres Selektivitätsindex I auf einer 17-C-Seitenkettensteroidverbindung derjenigen Art als Kohlenstoffquelle durchgeführt worden ist, die im Hauptverfahren als Einsatzmaterial zur Verwendung kommt, wobei vorzugsweise diese Steroidverbindung als einzige Kohlenstoffquelle zur Züchtung der Wildstämme eingesetzt worden ist.

24. Verfahren nach Ansprüchen 18 bis 23, dadurch gekennzeichnet, daß man mit solchen Defektblockmutantenstämmen arbeitet, die bei der Züchtung der Mutantenpopulation aus einer an sich bekannten Mutation der ausgewählten Wildstämme auf einem Mutanten-Trennmedium nicht oder praktisch nicht wachsen, während die unerwünschten begleitenden Mutantenstämme wachsen und hierdurch bzw. während ihres Wachstums abgetötet worden sind.

25. Verfahren nach Anspruch 24, dadurch gekennzeichnet, daß als Mutanten-Trennmedium ein wäßriges Nährmedium eingesetzt worden ist, das als Kohlenstoffquelle eine Steroidverbindung mit einer 17-C-Seitenkette mit bis zu 5 C-Atomen oder in 17-C-Stellung auch keine Seitenkette enthält, wobei jedoch vorzugsweise eine Steroidverbindung mit 3 C-Atomen in der 17-C-Seitenkette verwendet worden ist.

26. Verfahren nach Ansprüchen 24 und 25, dadurch gekennzeichnet, daß im Mutanten-Trennmedium als Kohlenstoffquelle wenigstens eine 17-C-Steroid-$\alpha$-propionsäureverbindung und diese dabei vorzungsweise als einzige Kohlenstoffquelle des Trennmediums eingesetzt worden ist.

27. Verfahren nach Ansprüchen 24 bis 26, dadurch gekennzeichnet, daß die als Verfahrensprodukt gewünchte(n) 17-C-Steroid-$\alpha$-propionsäureverbindung(en) als einzige Kohlenstoffquelle im Mutantentrennmedium eingesetzt worden sind.

28. Verfahren nach Ansprüchen 24 bis 27, dadurch gekennzeichnet, daß die auf dem Mutanten-Trennmedium wachsenden Stämme in Gegenwart der nicht wachsenden Defektblockmutanten und ohne deren Schädigung, z.B. durch Zusatz von Antibiotika oder unter Einsatz von $P^{32}$, abgetötet worden sind.

29. Verfahren nach Ansprüchen 18 bis 28, dadurch gekennzeichnet, daß man in Abwesenheit von $\alpha,\alpha'$-Dipyridyl oder 8-Hydroxychinolin und vorzugsweise auch in Abwesenheit von anderen das Wachstum und/oder den Steroidringabbau hemmenden Inhibitoren arbeitet.

30. Verfahren nach Ansprüchen 18 bis 29, dadurch gekennzeichnet, daß man mit Defektblockmutanten arbeitet, die im Inhibitor-freien Standardtest eine Ausbeute von $\Delta$-4 BNC und/oder $\Delta$-1,4 BNC auf Cholesterin oder Sitosterin von Wenigstens 30 Gew.-% und vorzugsweise auf Cholesterin von wenigstens 50 Gew.-%—bezogen auf eingesetzte Steroidverbindung—liefern.

31. Verfahren nach Ansprüchen 18 bis 30, dadurch gekennzeichnet, daß man mit Defektblockmutanten von Achromobacter, Arthrobacter, Bacillus, Brevibacterium, Corynebacterium, Flavobacterium, Microbacterium, Mycobacterium, Nocardia, Protaminobacterium, Serratia oder Streptomyces arbeitet.

32. Verfahren nach Ansprüchen 18 bis 31, dadurch gekennzeichnet, daß man mit einer Defektblockmutante des Wildstammes CBS 660.77 arbeitet.

33. Verfahren nach Ansprüchen 18 bis 32, dadurch gekennzeichnet, daß man mit der Defektblockmutante CBS 437.77 arbeitet.

34. Verfahren nach Ansprüchen 18 bis 33, dadurch gekennzeichnet, daß man mit Steroidverbindungen mit gesättigten und/oder ungesättigten 17-C-Seitenketten und vorzugsweise mit bis zu 10 C-Atomen, insbesondere mit 8 bis 10 C-Atomen arbeitet.

35. Verfahren nach Ansprüchen 18 bis 34 dadurch gekennzeichnet, daß man mit Sterinen tierischen und/oder pflanzlichen Ursprungs, insbesondere mit Cholesterin, Sitosterin, Stigmasterin, Campesterin und/oder Ergosterin oder ihren Abkömmlingen, wie Cholestenon, Sitostenon oder Stigmastenon arbeitet.

36. Verfahren nach Ansprüchen 18 bis 35, dadurch gekennzeichnet, daß man das zu transformierende Steroidsubstrat der Reaktionszone wenigstens weitgehend kontinuierlich zusetzt.

## Revendications

1. Procéde de préparation de micro-organismes mutants déficients bloqués capables d'assurer l'obtention, à l'échelle technique ou industrielle, de composés du type acide 17-C-stéroïde-alpha-propionique, en particulier pour la préparation de l'acide oxo-3 pregna-ène 4 carboxylique-20 (delta-4 BNC) et/ou de l'acide oxo-3 pregnadiène-1,4 carboxylique-20 (delta-1,4 BNC) à partir de substrats de stéroïdes comportant des chaînes latérales sur le carbone 17, par exemple à partir de stérols d'origine animale ou végétale, par dégradation des chaînes latérales s'effectuant au moins dans une large mesure de façon sélective même en l'absence d'inhibiteurs de la dégradation des noyaux de stéroïdes et/ou empêchant la croissance, dans des conditions aérobies, procédé caractérisé en ce que:

1) on isole et cultive une souche sauvage de microorganismes, capables d'une croissance aérobie sur des stérols constituant la seule source de carbone et l'on favorise ainsi une dégradation de la chaîne latérale en C—17 pultôt qu'une dégradation du noyau,

2) on soumet la souche sauvage à un traitement de mutation connu en soi,

3) on cultive la population des mutants sur un milieu séparateur sur lequel les mutants fournissant des composés du type acide 17-C-Stéroïdes-alpha-propioniques ne croissent pas ou pratiquement pas, cependant que les souches inopportunes accompagnant les mutants croissent et sont tuées ainsi ou pendant leur croissance, et

4) on cultive le reste des souches de mutants sur des stérols comportant une chaîne latérale sur le carbone en position 17 et l'on isole ainsi les souches assurant la production optimale des composés du type acid 17-C-stéroïde-alpha-propionique.

2. Procédé selon la revendication 1, caractérisé en ce qu'on répète les étapes opératoires de mutation 2) et de séparation des mutants 3) une ou plusieurs fois sur les souches de mutants encore capable de vivre (viables) provenant de la séparation 3) des mutants.

3. Procédé selon les revendications 1 et 2, caractérisé en ce qu'on choisit et cultive des souches sauvages qui, lors d'une croissance aérobie sur des stérols comportant sur le carbone en position 17 des radicaux alkyles saturés ou insaturés ayant 8 à 10 atomes de carbone, donnent dans des conditions normalisées une dégradation sélective selon la formule générale:

$$I = a \cdot 10^b$$

dans laquelle a représente le facteur de croissance et b le facteur de sélectivité de la croissance de la souche sauvage et l'indice I de sélectivité présente une valeur au moins égale à 10, de préférence au moins égale à 100 et en particulier une valeur d'au moins $10^5$.

4. Procédé selon les revendications 1 à 3, caractérisé en ce qu'on choisit et utilise des souches sauvages dont le facteur de sélectivité b vaut au moins 1, avantageusement au moins 2, et dont le facteur de croissance a vaut avantageusement au moins 0,2, en particulier également au moins 1, procédé dans lequel on peut en outre préférer considérer les souches sauvages tout d'abord d'après leur facteur de sélectivité b, pour des valeurs numériques aussi élevées que possible de b, et ensuite seulement pour leur facteur de croissance a.

5. Procédé selon les revendications 1 à 4, caractérisé en ce que la culture des souches sauvages s'effectue dans des conditions aérobies en présence d'un milieu nutritif aqueux comportant des stérols d'origine végétale ou animale, par exemple du cholestérol, du sitostérol, du stigma-stérol et/ou du campestérol, le stérol choisi servant de préférence de seule source de carbone pour la culture de la souche sauvage.

6. Procédé selon la revendication 5, caractérisé en ce que la culture des souches sauvages s'effectue déjà sur le stérol, constituant la source de carbone, et qui doit être dégradé, à l'aide des mutants déficients bloqués finalement obtenus, en un composé du type acide 17-C-stéroïde-alpha-propionique.

7. Procédé selon les revendications 1 à 6, caractérisé en ce qu'on effectue la mutation des souches sauvages choisies en utilisant un rayonnement à haute énergie ou par traitement avec des agents mutagènes, par exemple par traitement avec des nitrosoguanidines et/ou du méthanesulfonate d'éthyle.

8. Procédé selon les revendications 1 à 7, caractérisé en ce qu'on utilise, comme milieu séparateur pour une population de mutants se présentant lors de la mutation, un milieu nutritif aqueux qui contient comme source de carbone un stéroïde ayant sur le carbone en position 17 une chaîne latérale ayant jusqu'à 5 atomes de carbone ou, également sur le carbone en position 17, aucune chaîne latérale mais l'on utilise de préférence un stéroïde comportant 3 atomes de carbone dans la chaîne latérale fixée sur le carbone en position 17.

9. Procédé selon les revendications 1 à 8, caractérisé en ce qu'on utilise comme source de carbone dans le milieu de séparation des mutants au moins un composé de type acide 17-C-stéroïde-alpha-propionique, que l'on utilise alors avantageusement comme seule source de carbone ou tout au moins comme source quantitativement prédominante de carbone.

10. Procédé selon les revendications 1 à 9, caractérisé en ce qu'on utilise, dans le milieu de séparation des mutants, comme source de carbone les composés de type acide 17-C-stéroïde-alpha-propionique dont la préparation à l'aide des mutants déficients bloqués, cultivés selon l'invention, est finalement voulue.

11. Procédé selon les revendications 1 à 10, caractérisé en ce qu'on détruit les souches dont la croissance s'effectue sur le milieu séparateur des mutants en présence des mutants déficients bloqués qui n'y poussent pas ou pratiquement pas et qui ainsi ne subissent aucun dégât.

12. Procédé selon les revendications 1 à 11, caractérisé en ce qu'on effectue la destruction des souches dont la croissance s'effectue sur le milieu de séparation des mutants, par addition d'antibiotique, par exemple par addition d'une pénicilline ou par utilisation de composés comportant $P^{32}$.

13. Procédé selon les revendications 1 à 12, caractérisé en ce qu'on utilise, comme source au moins prédominante de carbone dans le milieu de séparation des mutants, l'acide oxo-3 pregnàne-4 carboxylique-20 et/ou l'acide oxo-3-pregnadiène-1,4 carboxylique-20.

14. Procédé selon les revendications 1 à 13, caractérisé en ce qu'on isole du milieu de séparation des mutants les souches de mutants encore viables et l'on en obtient les souches de mutants capables d'une productivité optimale du produit voulu dans le procédé de l'invention.

15. Procédé selon les revendications 1 à 14, caractérisé en ce qu'on travaille avec les souches sauvages de Achromobacter, Arthrobacter, Bacillus, Brevibacterium, Corynebacterium, Flavobacterium, Microbacterium, Mycobacterium, Nocardia, Protaminobacterium, Serratia ou Streptomyces.

16. Procédé selon les revendications 1 à 15, caractérisé en ce qu'on utilise la souche sauvage Corynebacterium CBS 660.77.

17. Nouvelle souche de micro-organismes mutants déficients bloqués CBS 437.77 ainsi que DSM 1435, 1437, 1439, 1442, 1443, 1444, 1445, qui conviennent pour une préparation sans inhibiteur de l'acide oxo-3 pregnène-4 carboxylique-20 et/ou de l'acide oxo-3 pregnadiène-1,4 carboxylique-20.

18. Procédé de préparation de l'acide oxo-3 pregnène-4 carboxylique-20 (delta-4 BNC) et/ou de l'acide oxo-3 pregnadiène-1,4 carboxylique-20 (delta-1,4 BNC) par coupure de dégradation microbienne de chaîne latérale sur des substrats de stéroïdes comportant une chaîne latérale sur le carbone en 17, procédé caractérisé en ce que:

a) on cultive, dans un milieu nutritif aqueux dans des conditions aérobies et en présence d'un substrat stéroïde comportant une chaîne latérale sur le carbone en 17, avec enrichissement en acide oxo-3 pregnène-4 carboxylique-20 et/ou en acide oxo-3 pregnadiène-1,4 carboxylique-20 un micro-organisme fournissant les delta-4 BNC et/ou delta-1,4 BNC avec un rendement d'au moins 15% en poids (par rapport au substrat stéroïde introduit), même en l'absence d'inhibiteurs destinés à empêcher la dégradation du noyau des stéroïdes et/ou la croissance, et

b) on isole ensuite l'acide oxo-3 pregnène-4 carboxylique-20 et/ou l'acide oxo-3 pregnadiène-1,4 carboxylique-20 formé(s).

19. Procédé selon la revendication 18, caractérisé en ce qu'on travaille avec, comme microorganismes, des lutants déficients bloqués qui ont été cultivés par mutation puis sélection à partir de souches sauvages choisies au préalable et qui peuvent croître sur des stéroïdes comportant des chaînes latérales sur le carbone en position 17, constituant avantageusement la seule source de carbone, en présentant, pour les chaînes latérales, approximativement la même vitesse de dégradation que pour la partie cyclique des stéroïdes, mais qui possèdent avantageusement une plus grande vitesse de dégradation des chaînes latérales.

20. Procédé selon les revendications 18 et 19, caractérisé en ce qu'on a cultivé les mutants déficients bloqués à partir de souches sauvages qui, lors d'une croissance sur des stéroïdes, donnent dans des conditions normalisées une dégradation sélective selon la formule générale:

$$I = a \cdot 10^b$$

dans laquelle a représente le facteur de croissance et b le facteur de sélectivité de la croissance de la souche sauvage, et l'indice de sélectivité I présente une valeur numérique au moins égale à 10, de préférence au moins égale à 100.

21. Procédé selon la revendication 20, caractérisé en ce que l'indice de sélectivité I de la souche sauvage est au moins égale à $10^5$.

22. Procédé selon les revendications 18 à 21, caractérisé en ce que le facteur b de sélectivité de la souche sauvage utilisée pour la culture du mutant déficient bloqué vaut au moins 2 et en ce que le facteur de croissance a vaut avantageusement au moins 0,2, en particulier aussi au moins 1.

23. Procédé selon les revendications 18 à 22, caractérisé en ce qu'on a effectué la culture des souches sauvages en vue d'une sélection subséquente selon leur indice I de sélectivité en utilisant comme source de carbone un stéroïde comportant une chaîne latérale sur le carbone en 17 et que l'on utilise dans le procédé principal comme matière de départ, ce stéroïde ayant avantageusement servi de seule source de carbone pour la culture de la souche sauvage.

24. Procédé selon les revendications 18 à 23, caractérisé en ce qu'on travaille avec des souches de mutants déficients bloqués qui, lors de la culture de la population des mutants provenant d'une mutation, connue en soi, des souches sauvages choisies, ne croissent pas ou pratiquement pas sur un

milieu de séparation des mutants cependant que les souches inopportunes accompagnant les mutants croissent et sont détruites ainsi ou pendant leur croissance.

25. Procédé selon la revendication 24, caractérisé en ce qu'on a utilisé comme milieu de séparation des mutants un milieu nutritif aqueux qui contient comme source de carbone un stéroïde ayant sur le carbone en 17 une chaîne latérale comportant jusqu'à 5 atomes de carbone ou ne comportant aucune chaîne latérale sur le carbone en position 17, mais l'on a utilisé de préférence un stéroïde comportant 3 atomes de carbone dans la chaîne latérale fixée sur le carbone en position 17.

26. Procédé selon les revendications 24 et 25, caractérisé en ce qu'on a utilisé dans le milieu de séparation des mutants, à titre de source de carbone, au moins un composé de type acide 17-C-stéroïde-alpha-propionique et l'on a utilisé ce composé de préférence comme seule source de carbone du milieu séparateur.

27. Procédé selon les revendications 24 à 26, caractérisé en ce qu'on a utilisé dans le milieu de séparation des mutants, comme seule souce de carbone, le ou les composés de type acide 17-C-stéroïde-alpha-propionique voulu(s) comme produit(s) de procédé.

28. Procédé selon les revendications 24 à 27, caractérisé en ce qu'on a tué, par exemple par addition d'antibiotiques ou en faisant agir $P^{32}$, les souches dont la croissance s'effectue sur le milieu de séparation des mutants en présence des mutants déficients bloqués qui ne croissent pas sur ce milieu et sans endommager ces derniers.

29. Procédé selon les revendications 18 à 28, caractérisé en ce qu'on travaille en l'absence d'alpha, alpha'-dipyridyle ou de l'hydroxy-8 quinoléine et avantageusement aussi en l'absence d'autres inhibiteurs qui bloquent la croissance et/ou la dégradation de noyaux de stéroïdes.

30. Procédé selon les revendications 18 à 29, caractérisé en ce qu'on travaille avec des mutants déficients bloqués qui dans un essai normal sans inhibiteur fournissent un rendement en delta-4 BNC et/ou delta-1,4 BNC, par rapport à du cholestérol ou à du sitostérol, d'au moins 30% en poids et avantageusement par rapport à du cholestérol d'au moins 50% en poids, par rapport au stéroïde utilisé.

31. Procédé selon les revendications 18 à 30, caractérisé en ce qu'on travaille avec des mutants déficients bloqués de Achromobacter, Arthrobacter, Bacillus, Brevibacterium, Corynebacterium, Flavobacterium, Microbacterium, Mycobacterium, Nocardia, Protaminobacterium, Serratia ou Streptomyces.

32. Procédé selon les revendications 18 à 31, caractérisé en ce qu'on travaille avec un mutant déficient bloqué de la souche sauvage CBS 660.77.

33. Procédé selon les revendications 18 à 32, caractérisé en ce qu'on travaille avec le mutant déficient bloqué CBS 437.77.

34. Procédé selon les revendications 18 à 33, caractérisé en ce qu'on travaille avec des stéroïdes comportant sur le carbone en position 17 des chaînes latérales saturées et/ou insaturées et ayant avantageusement jusqu'à 10 atomes de carbone, notamment 8 à 10 atomes de carbone.

35. Procédé selon les revendications 18 à 34, caractérisé en ce qu'on travaille avec des stérols d'origine animale et/ou végétale, notamment avec du cholestérol, du sitostérol du stigmastérol, du campestérol et/ou de l'ergostérol ou avec leurs dérivés comme la cholesténone, la sitosténone ou la stigmasténone.

36. Procédé selon les revendications 18 à 35, caractérisé en ce qu'on introduit dans la zone de réaction de façon au moins dans une large mesure, continue le stéroïde servant de substrat à transformer.

**Claims**

1. A process for producing microorganism defect block mutants capable of a technical scale production of 17-C-steroid-$\alpha$-propionic acid compounds, more specifically the production of 3-oxo-pregna-4-ene-20-carboxylic acid ($\Delta$-4 BNC) and/or 3-oxo-pregna-1,4-diene-20-carboxylic acid ($\Delta$-1,4 BNC) from 17-C side chain steroid substrates, e.g. from steroid compounds of animal or vegetable origin by an at least far-reachingly selective side chain degradation even in the absence of inhibitors inhibiting the steroid ring degradation and/or the growth under aerobic conditions, characterised in that
1) a microorganism wild strain is isolated and cultivated which strain is cabable of an aerobic growth on steroid compounds as the sole carbon source and therein prefers the 17—C side chain degradation to a ring degradation;
2) the wild strain is subjected to a *per se* known mutation treatment;
3) the population of mutants is cultivated on a separating medium on which the mutants yielding 17—C— steroid-$\alpha$-propionic acid compounds do not grow or do substantially not grow, while the undesired accompanying mutant strains do grow and are killed hereby or in the course of the growth thereof; and
4) the remaining residue of the mutant strains are cultivated to provide 17—C side chain steroid compounds and in this procedure those strains exhibiting an optimum production of 17-C-steroid-$\alpha$-propionic acid compounds are recovered.

2. The process according to claim 1, characterized in that the process steps of the mutation 2) and the mutant separation 3) are repeated one or more times with the still viable mutant strains from the mutant separation 3).

3. The process according to claims 1 and 2, characterized in that wild strains are selected and cultivated which, during aerobic growth on steroid compounds having saturated or unsaturated alkyl residues in the 17—C position of from 8 to 10 carbon atoms, yield a selective degradation effect according to the general formula

$$I = a \cdot 10^{b}$$

wherein *a* is the growth factor and *b* is the selectivity factor of the wild strain growth, and the selectivity index I has a numerical value of at least 10, and preferably at least 100, and, more specifically, of at least $10^5$.

4. The process according to claims 1 to 3, characterized in that wild strains are selected and cultivated the selectivity factor *b* of which it as least 1, and preferably at least 2, and the growth factor *a* of which is preferably at least 0.2, and, more specifically, also at least 1, wherein it may further be preferred to first consider the wild strains with respect to their selectivity factors *b*—with as high as possible numerical values of *b*—and only then with respect to their growth factors *a*.

5. The process according to claims 1 to 4, characterized in that the cultivation of the wild strains is carried out under aerobic conditions in the presence of an aqueous nutrient medium on steroids of vegetable or animal origin such as cholesterol, sitosterol, stigmasterol and/or campesterol, wherein it is preferred to employ the selected steroid compound as the only carbon source for the cultivation of the wild strains.

6. The process according to claim 5, characterized in that already the cultivation of the wild strains is carried out on that steroid compound as carbon source which is to be degraded by the eventually obtained defect block mutant to give the 17-C-steroid-$\alpha$-propionic acid compound.

7. The process according to claims 1 to 6, characterized in that the mutation of the selected wild strains is carried out with an energy-rich radiation or by a treatment with mutagenic agents, i.e. by a treatment with nitrosoguanidines and/or ethylmethanesulfonate.

8. The process according to claims 1 to 7, characterized in that as the separating medium for a population of mutants having been obtained in the mutation an aqueous nutrient medium is employed which has, as the carbon source, a steroid compound having a 17—C side chain with up to 5 carbon atoms or no side chain in the 17—C position, with a steroid compound having 3 carbon atoms in the 17—C side chain being preferredly used.

9. The process according to claims 1 to 8, characterized in that as the carbon source in the mutants-separating medium a 17-C-steroid-$\alpha$-propionic acid compound is employed which therein is preferably used as the only carbon source or at least as the predominant carbon source with respect to the amount thereof.

10. The process according to claims 1 to 9, characterized in that in the mutants-separating medium as the carbon source those 17-C-steroid-$\alpha$-propionic acid compounds are employed the production of which is eventually desired by means of the defect block mutants as cultivated according to the invention.

11. The process according to claims 1 to 10, characterized in that the strains growing on the mutants-separating medium are killed in the presence of those defect block mutants which do not grow or do substantially not grow and without damaging same.

12. The process according to claims 1 to 11, characterized in that killing the strains growing in the mutants-separating medium is effected by the addition of an antibiotic, for example by addition of a penicillin compound or by the combined use of $P^{32}$ compounds.

13. The process according to claims 1 to 12, characterized in that 3-oxo-pregna-4-ene-20-carboxylic acid and/or 3-oxo-pregna-1,4-diene-20-carboxylic acid is/are used as the at least predominant carbon source in the mutants-separating medium.

14. The process according to claims 1 to 13, characterized in that the still viable mutant strains are isolated from the mutants-separating medium and the mutant strains having an optimum productivity of the desired process product are recovered therefrom.

15. The process according to claims 1 to 14, characterized in that wild strains of *Achromobacter, Arthrobacter, Bacillus, Brevibacterium, Corynebacterium, Flavobacterium, Microbacterium, Mycobacterium, Nocardia, Protaminobacterium, Serratia* or *Streptomyces* are employed.

16. The process according to claims 1 to 15, characterized in that the wild strain *Corynebacterium* CBS 660.77 is employed.

17. New defect block mutant strains CBS 437.77 as well as DSM 1435, 1437, 1439, 1442, 1443, 1444, 1445 which are suitable the inhibitor-free production of 3-oxo-pregna-4-ene-20-carboxylic acid and/or 3-oxo-pregna-1,4-diene-20-carboxylic acid.

18. A process for the production of 3-oxo-pregna-4-ene-20-carboxylic acid ($\Delta$-4 BNC) and/or 3-oxo-pregna-1,4-diene-20-carboxylic acid ($\Delta$-1,4 BNC) by the microbial side chain degradiation at 17—C side chain steroid substrates, characterized in that

a) a microorganism, which produces $\Delta$-4 BNC and/or $\Delta$-1,4 BNC in a yield of at least 15% by weight—based on the starting steroid substrate—even in the absence of inhibitors for inhibiting the steroid ring degradation and or the growth, is cultivated in an aqueous medium under aerobic

conditions and in the presence of a 17—C side chain steroid substrate to give an enrichment in 3-oxo-pregna-4-ene-20-carboxylic acid and/or 3-oxo-pregna-1,4-diene-20-carboxylic acid; and

b) the 3-oxo-pregna-4-ene-20-carboxylic acid and/or 3-oxo-pregna-1,4-diene-20-carboxylic acid having been formed is/are subsequently isolated.

19. The process according to claim 18, characterized in that defect block mutants are employed as the microorganisms which mutants have been cultivated by mutation and subsequent selection from those previously selected wild strains that are capable of growing on steroid compounds having 17—C side chains as preferably the only carbon source with at least the same degradation rate for the side chains as for the ring portion of the steroid compounds, while however, those having an increased side chain degradation rate are preferred.

20. The process according to claims 18 and 19, characterized in that in the employed defect block mutants have been cultivated from wild strains which, in the growth on steroid compounds, yield a selective degradation effect according to the general formula

$$I = a \cdot 10^b$$

wherein *a* is the growth factor and *b* is the selectivity factor of the wild strain growth, and the selectivity index I has a numerical value of at least 10, and preferably at least 100.

21. The process according to claim 20, characterized in that the selectivity index I of the wild strain is at least $10^5$.

22. The process according to claims 18 to 21, characterized in that the selectivity factor *b* of the wild strain employed for the cultivation of the defect block mutant is at least 2, and the growth factor *a* is preferably at least 0.2, and, more specifically, also at least 1.

23. The process according to claims 18 to 22, characterized in that the cultivation of the wild strains for the subsequent selection according to their selectivity index I has been carried out on a 17—C side chain steroid compound as the carbon source of that type which is employed as feedstock in the main process, said steroid compound having been preferably used as the only carbon source for the cultivation of the wild strains.

24. The process according to claims 18 to 23, characterized in that those defect block mutants are employed that, in the cultivation of the population of mutants from a *per se* known mutation of the selected wild strains, do not grow or do substantially not grow on a mutants-separating medium, while the undesired accompanying mutant strains do grow and have been killed hereby and/or during their growth, respectively.

25. The process according to claim 24, characterized in that, as the mutants-separating medium, an aqueous nutrient medium has been employed which contains, as the carbon source, a steroid compound having a C—17 side chain with up to 5 carbon atoms or no side chain in the 17—C position, with a steroid compound having 3 carbon atoms in the 17—C side chain having been preferredly used.

26. The process according to claims 24 and 25, characterized in that as the carbon source in the mutants-separating medium at least one 17-C-steroid-$\alpha$-propionic acid compound has been employed which preferably has been used as the only carbon source.

27. The process according to claims 24 to 26, characterized in that the 17-C-steroid-$\alpha$-propionic acid compound(s) desired as the process product(s) has/have been employed as the only carbon source in the mutants-separating medium.

28. The process according to claims 24 to 27, characterized in that the strains growing on the mutants-separating medium have been killed in the presence of the defect block mutants which do not grow and without damage to same, e.g. by the addition of antibiotics or by use of $P^{32}$.

29. The process according to claims 18 to 28, characterized in that the process is carried out in the absence of $\alpha,\alpha'$-dipyridyl or 8-hydroxyquinoline and preferably also in the absence of other inhibitors inhibiting the growth and/or steroid ring degradation.

30. The process according to claims 18 to 29, characterized in that defect block mutants are employed which, in the inhibitor-free standard test on cholesterol or sitosterol, give a yield of $\Delta$-4 BNC and/or $\Delta$-1,4 BNC of at least 30% by weight, and preferably on cholesterol of 50%—based on the starting steroid compound.

31. The process according to claims 18 to 30, characterized in that defect block mutants of *Achromobacter, Arthrobacter, Bacillus, Brevi-bacterium, Corynebacterium, Flavobacterium, Microbacterium, Mycobacterium, Nocardia, Protaminobacterium, Serratia* or *Streptomyces* are employed.

32. The process according to claims 18 to 31, characterized in that a defect block mutant of the wild strain *Corynebacterium* CBS 660.77 is employed.

33. The process according to claims 18 to 32, characterized in that the defect block mutant CBS 437.77 is employed.

34. The process according to claims 18 to 33, characterized in that steroid compounds having saturated or unsaturated 17—C side chains, and preferably those having up to 10 carbon atoms, and more particularly those having from 8 to 10 carbon atoms, are employed.

35. The process according to claims 18 to 34, characterized in that steroids of animal and/or vegetable origin are employed, preferably cholesterol, sitosterol, stigmasterol, campesterol and/or ergosterol or the derivatives thereof such as cholestenone, sitostenone or stigmastenone.

36. The process according to claims 18 to 35, characterized in that the steroid substrate to be transformed is at least far-reachingly continuously added to the reaction zone.